(19) Europäisches Patentamt
European Patent Office
Office européen des brevets

(11) Publication number: **0 169 410 B1**

(12) **EUROPEAN PATENT SPECIFICATION**

(45) Date of publication of patent specification: **24.02.93**

(51) Int. Cl.[5]: **C07D 487/04**, C07D 519/00, C07F 9/58, A61K 31/40, //(C07D487/04,209:00,205:00), (C07D519/00,491:00,487:00), (C07D519/00,495:00,487:00), (C07D519/00,487:00,471:00), (C07D519/00,487:00,455:00), (C07D519/00,487:00,453:00)

(21) Application number: **85108133.1**

(22) Date of filing: **01.07.85**

The file contains technical information submitted after the application was filed and not included in this specification

(54) **Carbapenems having a 2-quaternary pyridine altylthio or pyridine alkenylthio substituent, compositions containing the same and combinations with DHP inhibitors.**

(30) Priority: **02.07.84 US 626580**

(43) Date of publication of application: **29.01.86 Bulletin 86/05**

(45) Publication of the grant of the patent: **24.02.93 Bulletin 93/08**

(84) Designated Contracting States: **AT BE CH DE FR GB IT LI LU NL SE**

(56) References cited:
**EP-A- 0 044 170**
**FR-A- 2 524 890**
**GB-A- 2 136 802**

(73) Proprietor: **MERCK & CO. INC.**
**126, East Lincoln Avenue P.O. Box 2000**
**Rahway New Jersey 07065-0900(US)**

(72) Inventor: **Christensen, Burton G.**
**770 Anderson Avenue, Apt. 19H**
**Cliffside Park New Jersey 07010(US)**
Inventor: **Johnston, David B. R.**
**53 Round Top Road**
**Warren New Jersey 07060(US)**
Inventor: **Schmitt, Susan M.**
**50 M Southwyck Village**
**Scotch Plains New Jersey 07076(US)**

(74) Representative: **Abitz, Walter, Dr.-Ing. et al**
**Abitz, Morf, Gritschneder, Freiherr von Wittgenstein Postfach 86 01 09**
**W-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

The present invention is concerned with improved carbapenem antibiotics characterized by having a quaternary pyridinealkylthio or pyridinealkenylthio substituent in the 2-position.

Thienamycin is a known carbapenem, broad spectrum antibiotic of the formula:

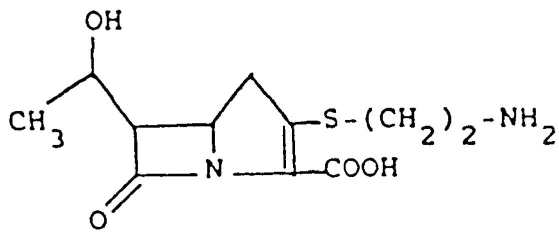

A

Other derivatives of A are also known. For example, FR-A-2524890 discloses derivation of A in which the nitrogen of the amino group is part of a quaternized heteroarylium group. The present quaternary pyridine alkyl or alkenyl thio substituted carbapenems have an antibiotic spectrum equal to or better than A. The present carbapenems also are more resistant than A to degradation by the dehydropeptidase enzyme DHP-I.

SUMMARY OF THE INVENTION

Carbapenems having the formula

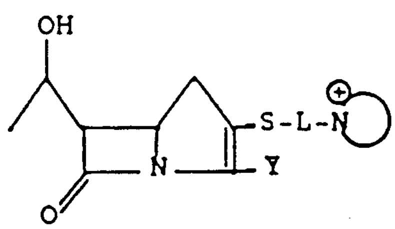

where

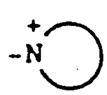

is a quaternary pyridine group and their use as antibiotics.

DETIAILED DESCRIPTION OF THE INVENTION

An embodiment of the present invention is a compound having the formula

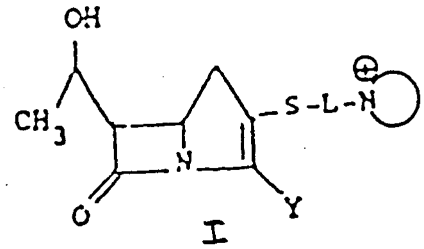

I

wherein 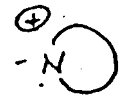 is 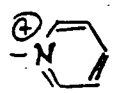 and

wherein the pyridinium group is required to be substituted by:

A: an acidic side-chain of the structure A or $-(CH_2)_n-X-(CH_2)_m-Y'-A$ where:

- n = 0 - 4
- m = 0 - 4
- X = $CHR^S$, CH=CH, phenylene ($-6_6H_4-$), NH, N($C_1$-$C_4$ alkyl), O, S, S=O, C=O, $SO_2$, $SO_2NH$, $CO_2$, CONH, $OCO_2$, OC=O, NHC=O;
- $R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);
- Y' = single bond, NH, N($C_1$-$C_4$ alkyl), O, S;
- A = an acidic function selected from carboxy ($CO_2H$), phosphono [$P=O(OH)_2$], alkylphosphono {P=O(OH)-[O($C_1$-$C_4$ alkyl)]}, alkylphosphinyl (P=O(OH)-($C_1$-$C_4$ alkyl)], phosphoramido [P=O(OH)-NH($C_1$-$C_4$ alkyl) and $P=O(OH)NHR^x$], sulfino ($SO_2H$), sulfo ($SO_3H$), 5-tetrazolyl ($CN_4H$), heteroarylsulfonamido ($SO_2NHR^x$) and acylsulfonamides represented by the structures $CONHSO_2$($C_1$-$C_4$ alkyl), $CONHSO_2N$($C_1$-$C_4$ alkyl)$_2$, $SO_2NHCO$($C_1$-$C_4$ alkyl) and $SO_2NHCOR^x$;
- $R^x$ = monocyclic or bicyclic heteroaryl group containing (a) when monocyclic, up to 3 heteroatoms and up to 6 total ring atoms or (b) when bicyclic up to 5 heteroatoms and 9-10 ring atoms in which the heteroatoms are selected from nitrogen, oxygen and sulfur;

and optionally by

B: a halogen atom;

L is a straight or branched chain, bivalent $C_1$-$C_6$ alkylene, $C_2$-$C_6$ alkenylene or $C_3$-$C_6$ cycloalkylene, or -$C_1$-$C_4$ alkylene-X-$C_1$-$C_4$ alkylene wherein X is O, S, NH, or N($C_1$-$C_4$ alkyl);

and

Y is

i) -COOH, a pharmaceutically acceptable ester or salt thereof;
ii) COOR wherein R is a removable carboxy protecting group;
iii) COOM wherein M is an alkali metal; or
iv) $COO^-$ provided that when Y is other than iv) a counterion $Z^{\ominus}$ is present.

Preferably the pyridinium group is substituted by -COOH, $-SO_3H$, $-C_{1-6}$alkylene-COOH, or $-C_{1-6}$alkylene $-SO_3H$.

As used herein, the term "heteroatom" means nitrogen, oxygen, or sulfur, independently selected where more than one heteroatom is involved.

A preferred group of compounds of Formula I are those where L is $C_1$-$C_6$ branched or linear alkylene.

Examples of preferred L groups are

$-CH_2-$, $-CH(CH_3)-$, $-CH(CH_2CH_3)-$, $-CH_2-CH_2-$, $-CH(CH_3)-CH_2--CH(CH_2CH_3)-CH_2-$, $-C(CH_3)_2-CH_2-$, $-(CH_2)_3-$, $-CH(CH_3)-(CH_2)_2-$, $-CH_2-CH(CH_3)-$, $-CH_2-C(CH_3)_2-CH_2-$, or $-(CH_2)_4-$.

Especially preferred compounds of Formula 1 are those where L is $-CH_2-$, $-CH_2CH_2-$or $-CH(CH_3)-CH_2$. Of course it is understood that where any substituent group has an asymmetric center e.g.

$$-\overset{CH_3}{\overset{|}{C}H}-CH_2-,$$

then all stereoisomers are included as mixtures or as separate isomers.

Of particular interest and the most preferred group are compounds of the present invention with the acidic function as defined above and the Y substituent in the 3-position being $COO^{\ominus}$ as defined above, thus forming a zwitterion with the positive charge of the quaternary nitrogen. The acidic function is anionic and the compounds are thus anionic zwitterions, i.e., they have a net negative charge. This novel characteristic has been found to result in at least one surprising and important improvement in the biological properties of the compounds ; reduced CNS side-effects. A more particular group of the compounds, that wherein the acidic function is a sulfoalkyl group of the formula $(C_{1-4}\ alkyl)SO_3^{\ominus}$, has been found to have the additional surprising and important biological property of enhanced potency against Pseudomonas species, an especially important nosocomial pathogen.

Representative examples of preferred

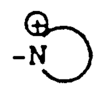

pyridinium groups are those having the formulae

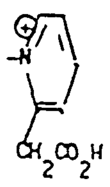
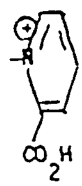
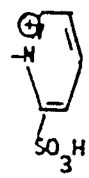
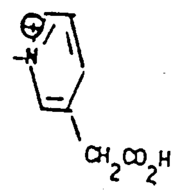

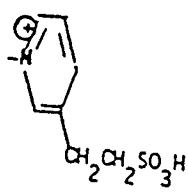
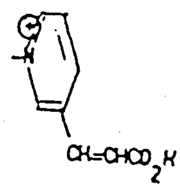
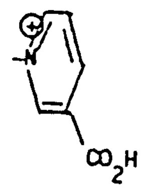
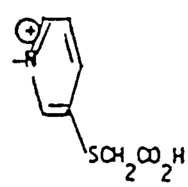

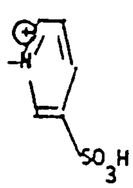
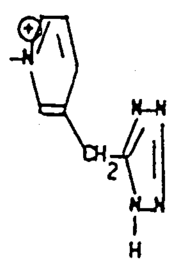
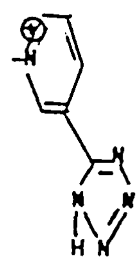
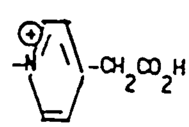

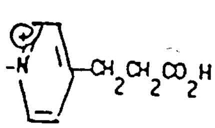
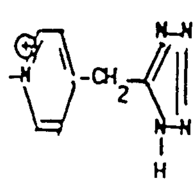
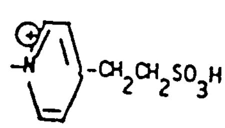

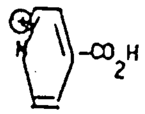

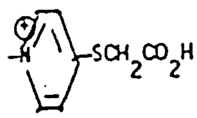

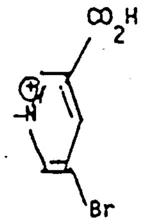

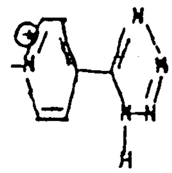

The following compounds are preferred:

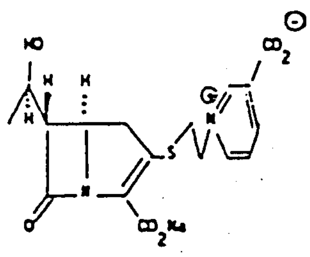

)

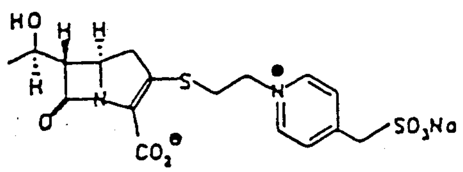

)

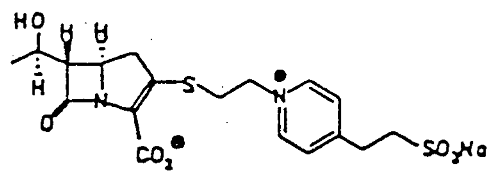

and

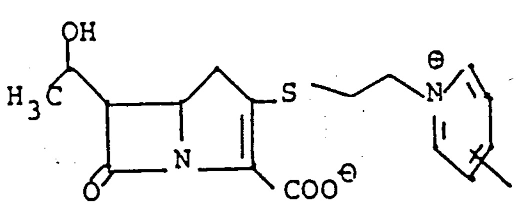

A

wherein

A is an acidic side-chain of the structure $-(CH_2)_n-X-(CH_2)_m-Y'-A$ as previously defined.

A further embodiment of the present invention is a compound having the formula:

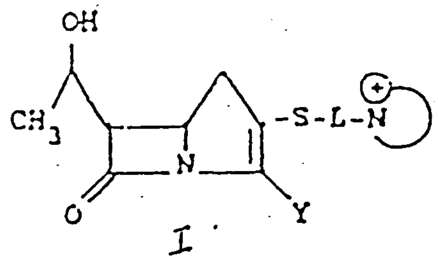

I

wherein L and Y are as previously defined and

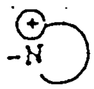 is 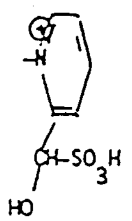, 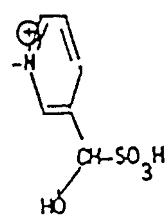, 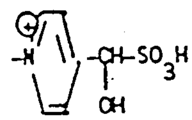,

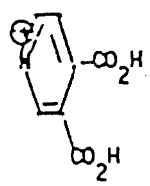 or 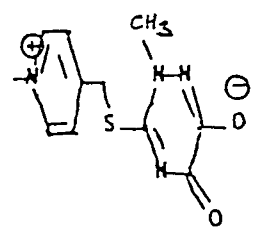.

Preferred compounds have the formula:

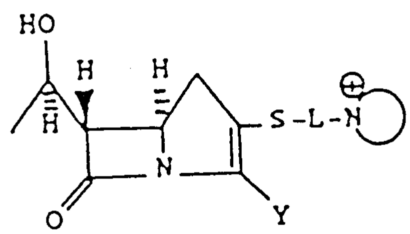

I

wherein L is $-CH_2CH_2-$, Y is $-CO_2Na$ and

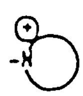

is

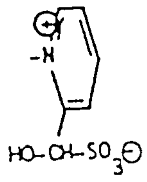, 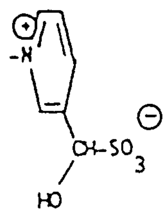, 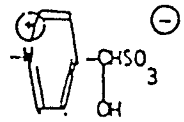 or 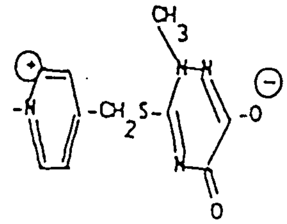

The compounds of Formula I include inner (Zwitterion) salts when Y is $COO^{\ominus}$ e.g.

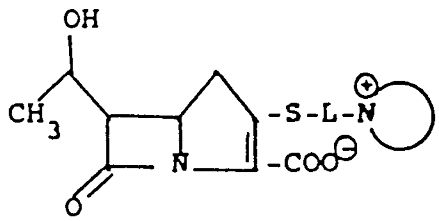

or, when Y is other than $COO^{\ominus}$, salts with an external, physiologically acceptable counterion $Z^{\ominus}$ e.g.

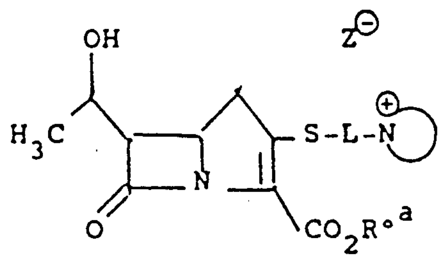

$R^{\circ a}$ is a pharmaceutically acceptable ester, e.g., pivaloyloxymethyl, phthalidyl, phthalimidomethyl, acetoxymethyl, ethoxycarbonyloxyethyl, pivaloyloxyethyl, 4-methyl-2-oxo-1,3-dioxolen-5-yl-methyl or salt group; and $Z^{\ominus}$ is, e.g., $Cl^{\ominus}$, $Br^{\ominus}$, $I^{\ominus}$, $OH^{\ominus}$, $HCO_3^{\ominus}$, or $CH_3CO_2^{\ominus}$. The inner salts are preferred.

Again, the compounds of Formula I include the stereoisomers as mixtures and as separate isomers. Compounds having the (5R,6S,8R) stereochemistry shown below are preferred

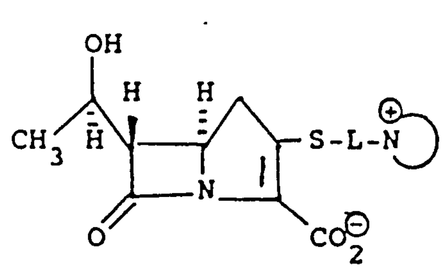

When L contains a chiral center, the side chain chirality leads to diastereomeric products. The products can be separated by conventional methods, used as mixtures or synthesized stereospecifically from optically active mercaptans.

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella

pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the formula I antibiotic is parenteral by i.v. infusion, i.v. bolus. or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d.) three (t.i.d.) or four (q.i.d.) times per day. More specifically, for mild infections, and particularly urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102616.4 filed July 24, 1979 (patent no. 10573); 79102615.6, filed July 24, 1979 (application no. 15573); and No. 82107174.3. filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.) disclose suitable inhibitors and combination compositions they are incorporated herein by reference. A preferred weight ratio of I compound:DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and pharmaceutical compositions for antibiotic use comprising the combination compositions and, optionally, a pharmaceutically acceptable carrier are further embodiments of the present invention.

The compounds of Formula I may be prepared by any convenient process.

One such process is illustrated by the following reaction equations:

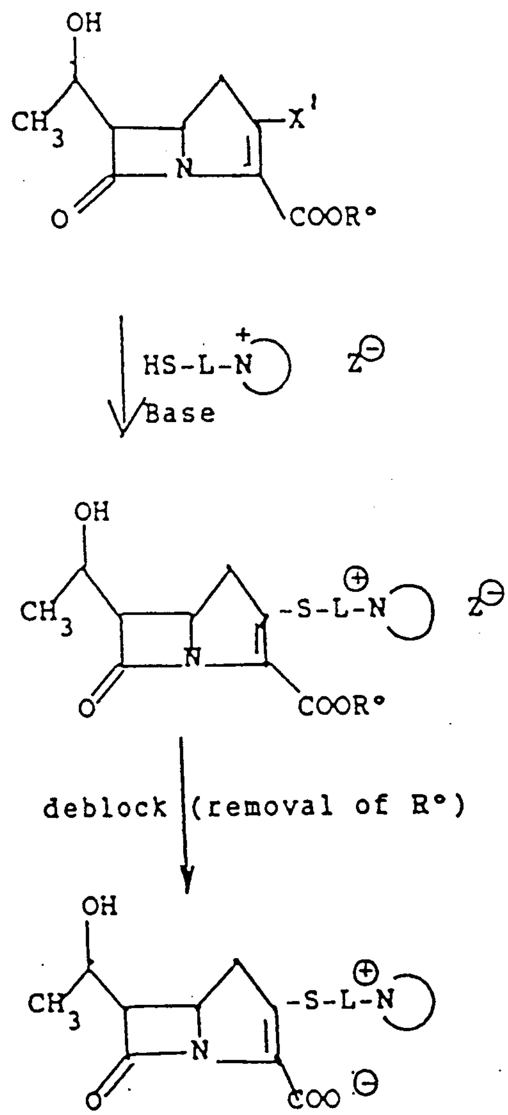


where R° is other than (-) or H and is a readily removable blocking group e.g. p-$NO_2$benzyl or allyl. X' is a leaving group such as OP(O)(OØ)$_2$,

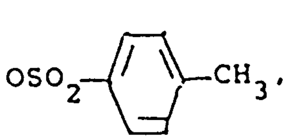,
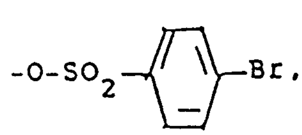

$OSO_2CF_3$, S(O)R" where R" is $CH_3$, $CH_2CH_3$, or $CH_2CH_2NHC(O)CH_3$ and $Z^{\ominus}$ is an anion such as $Cl^{\ominus}$, $Br^{\ominus}$, $I^{\ominus}$, $CH_3CO_2^{\ominus}$, $CH_3SO_3^{\ominus}$, $CF_3SO_3^{\ominus}$.

The process conditions are generally those available in the prior art. Thus, the side chain addition reaction is carried out in a solvent such as acetonitrile, dimethylformamide, dimethylacetamide, or N-ethylpyrrolidinone in the presence of a base such as N,N-diisopropylethylamine, triethylamine, or 4-dimethylaminopyridine at a temperature of from -40°C to 25°C for a period of 5 minutes to 10 hours. The

deblocking reaction wherein R° is p-nitrobenzyl is usually conducted in an aqueous system containing cosolvents such as tetrahydrofuran, ethanol, n-butanol, 2-amyl alcohol, or ethyl acetate and a pH 6.8-7.0 aqueous buffer. Suitable buffers include phosphate buffers and buffers derived from non-nucleophilic amines such as N-methylmorpholine or morpholinopropane sulfonic acid. The reaction is conducted at 0 °C to 40 °C for 0.5 to 5 hours under 1-100 atmospheres of hydrogen in the presence of a catalyst such as 10% palladium on carbon or 20% palladium hydroxide on carbon. The final products are purified by ion exchange chromatography and/or reverse phase chromatography. When a pharmaceutically acceptable ester of the final product is desired, the deblocking step is omitted and the appropriate R° group is incorporated into the starting material.

A second process for preparing Formula I compounds is illustrated by the following reaction equations:

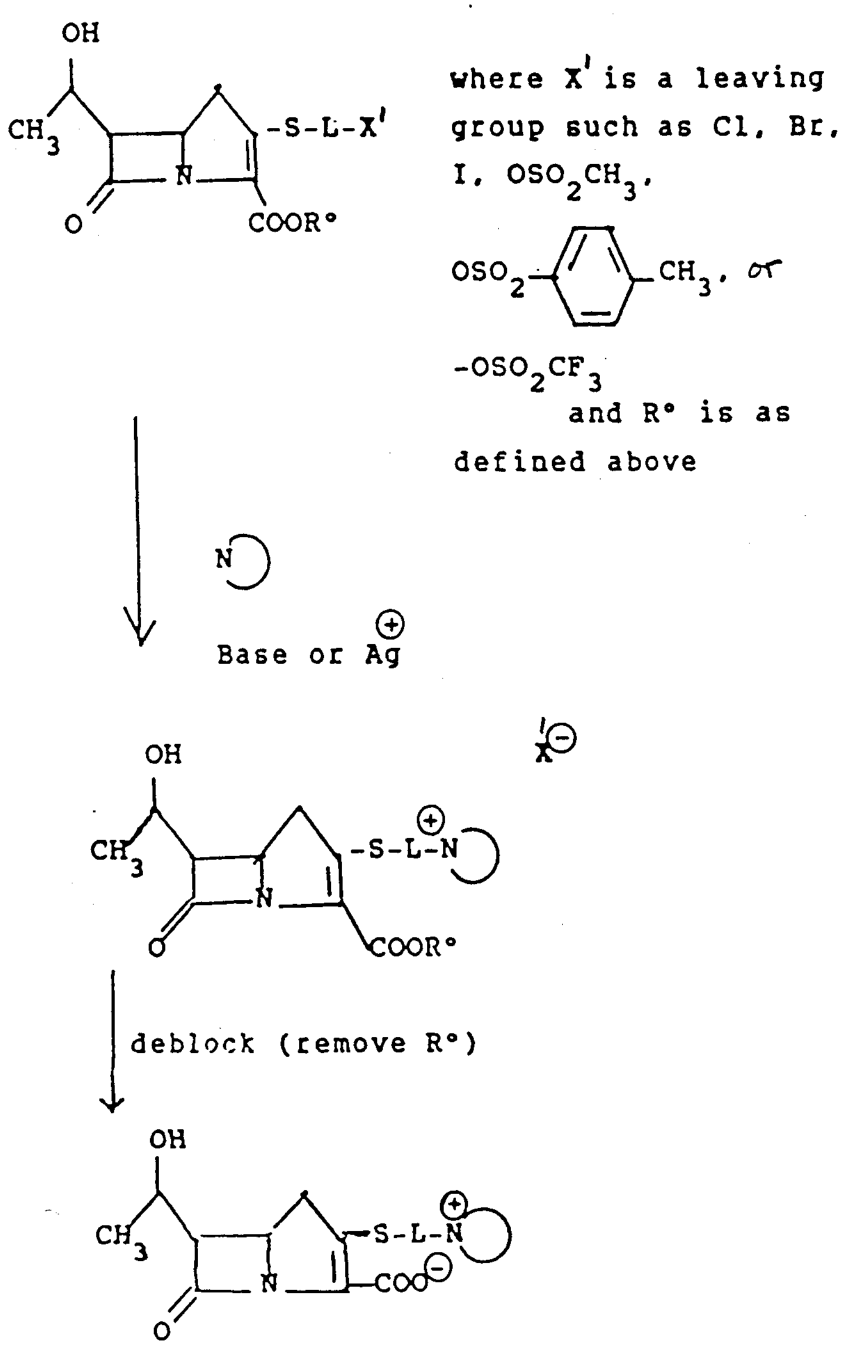


Again, the process conditions are those available in the prior art.

A third process for preparing Formula I compounds is illustrated by the following equations:

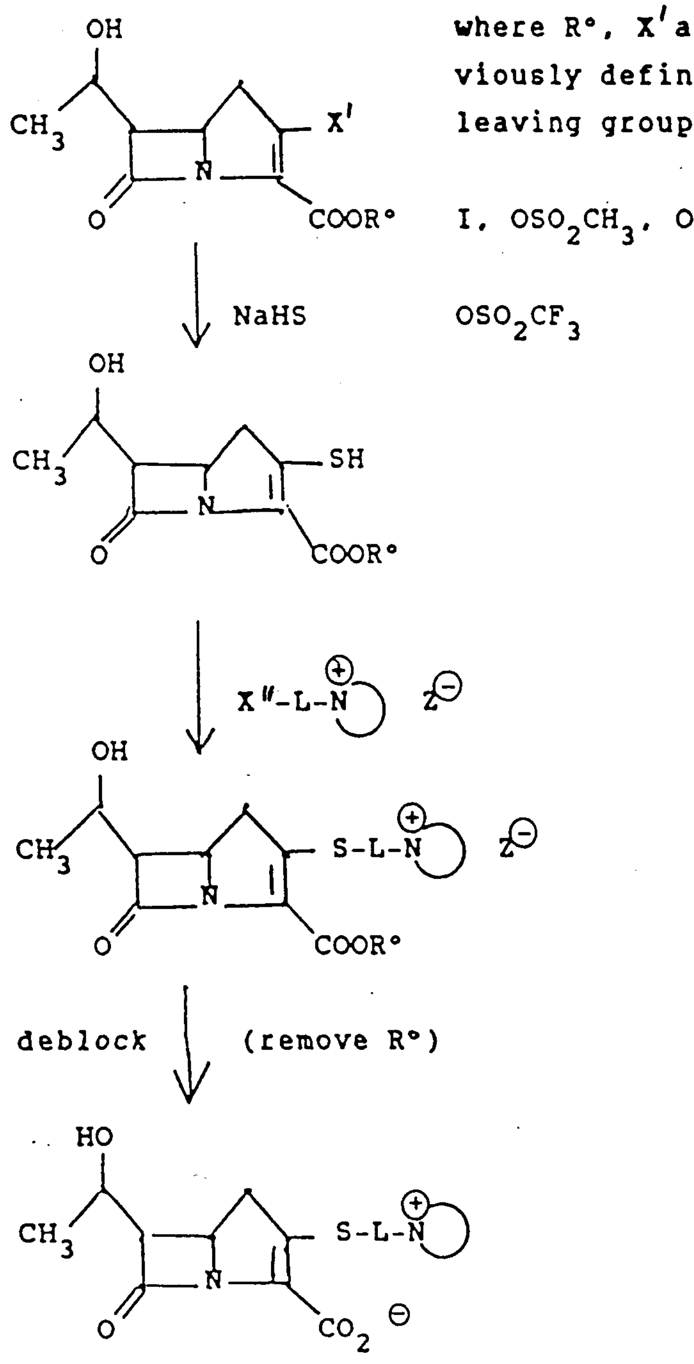


where R°, X' and Z are as previously defined and X'' is a leaving group such as Cl, Br, I, $OSO_2CH_3$, $OSO_2$-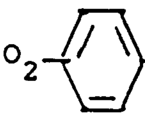-$CH_3$, or $OSO_2CF_3$.

Again, the process conditions are those available in the prior art.

Following are examples illustrating the preparation of compounds of Formula I. Temperatures are in °C unless otherwise specified.

EXAMPLE 1

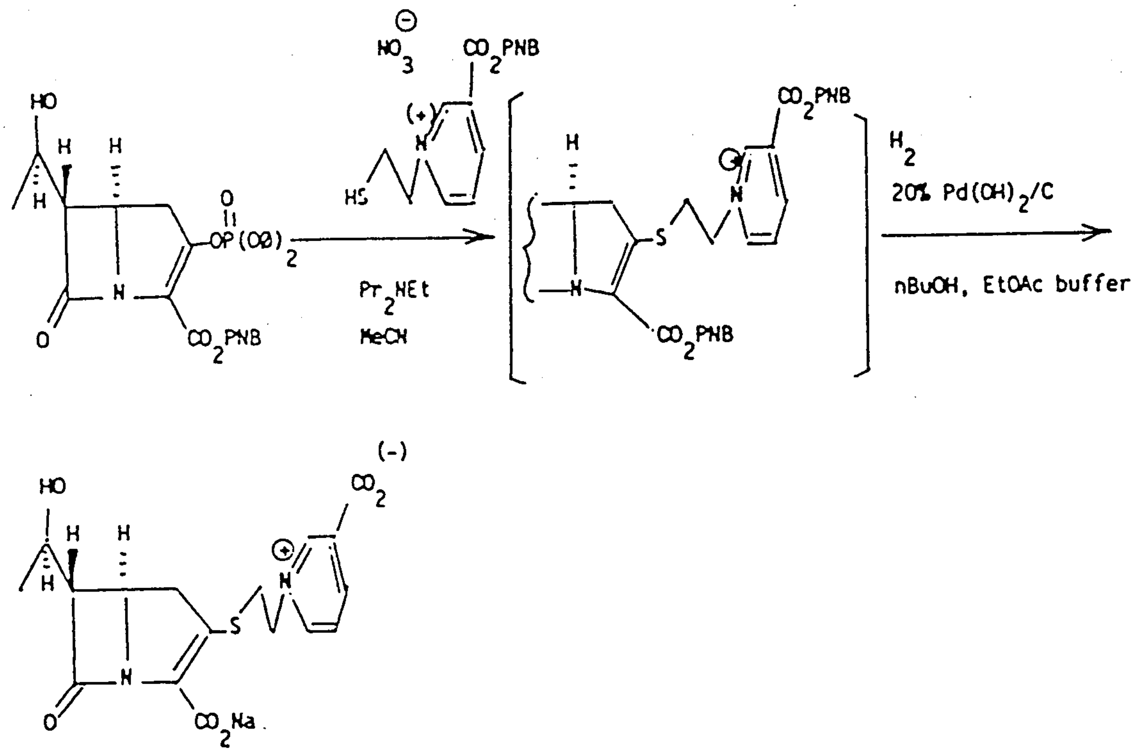


Sodium (5R,6S)-2-[2-(3-carboxylatopyridinium)ethyl] thio-6-[1(R)-hydroxyethyl]carbapen-2-em-3-carboxylate

A solution of p-nitrobenzyl (5R,6S)-2-(diphenylphosphono)oxy-6-[1(R)-hydroxyethyl]carbapen-2-em-3-carboxylate (175 mg, 0.30 mmol) and 1-(2-mercaptoethyl)-3-(p-nitrobenzyloxycarbonyl)-pyridinium nitrate (120 mg, 0.32 mmol) in anhydrous acetonitrile (3.0 ml) was cooled in an ice-bath under a nitrogen atmosphere and treated with N,N-diisopropylethylamine (0.11 ml, 0.63 mmol). The resulting solution was left at ice-bath temperature overnight, then diluted with diethylether (30 ml) and passed through a celite® pad. The ether insoluble oil and the residue left on the celite® pad were dissolved in a mixture of n-butanol (19 ml), ethylacetate (9 ml), water (19 ml), and 0.5M pH 6.8 N-methyl morpholine-hydrochloric acid buffer (9 ml), treated with 20% palladium hydroxide on carbon (105 mg), and hydrogenated on a Parr® shaker at 2.76 bar (40 psi) for 1 hour. The mixture was centrifuged to remove the catalyst and the supernatant was washed with methylene chloride (2 X 25 ml). The aqeuous phase was concentrated under vacuum and added to a column of Dowex® 50W-X4 resin (sodium form, 0.037 - 0.074 mm (200-400 mesh), 1.5 X 33 cm) which was eluted with deionized water in a cold room. The product containing fractions were located by UV, combined, and concentrated under vacuum to ca. 2.5 ml. The solution was applied to four Analtech 0.5 mm X 20 X 20 cm RPS-F plates which were developed with water in a cold room. The major UV visible band was removed and eluted with 4:1 acetonitrile-water. The extracts were washed with hexane, concentrated under vacuum, and lyophilized to provide the title compound (60.5 mg) as an amorphous, yellow solid.

IR (Nujol)$\nu$max 3350, 1750, 1645, 1605, 1580 $cm^{-1}$;
UV ($H_2O$)$\lambda$max 296 nm ($\epsilon$ 6,970); ($H_2O$ + $NH_2OH$) extinguished $\lambda$max 297 nm ($\epsilon$ 6,250);
NMR ($D_2O$) $\delta$ 1.27 (d. J = 6.4 Hz, $CH_3$ CHOH), 2.94 (dd, J = 8.8 and 17.5 Hz, CHaHb), 3.11 (dd, J = 9.7 and 17.5 Hz, CHaHb), 3.33 (dd, J = 2.7 and 6.2 Hz, H6), 3.40 (td, J = 6.2 and 15.2 Hz, SCHaHb), 3.58 (td, J = 6.3 and 15.2 Hz, SCHaHb), 4.01 (dt, J = 2.5 and 9.1 Hz, H5), 4.20 (~ p, J = 6 Hz, $CH_3$CHOH), 4.8-4.9 (m, $NCH_2$), 8.10 (dd, ArH), 8.90-8.95 (m, 2 ArH), 9.26 (m, ArH).

The following examples illustrate preparation of useful intermediates for the preparation of Formula I compounds.

EXAMPLE 2

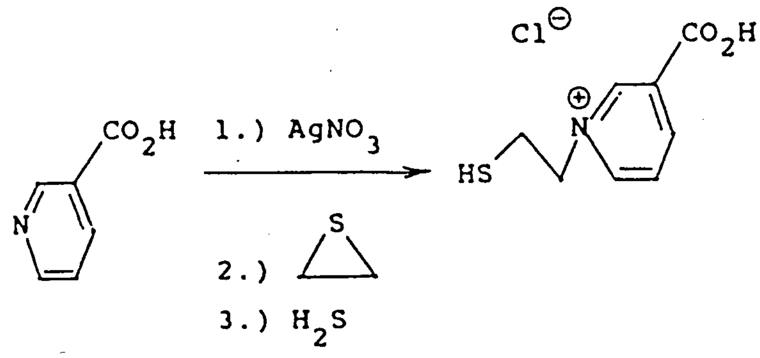


3-Carboxy-1-(2-mercaptoethyl)pyridinium chloride

Silver nitrate (4.14 g, 24.4 mmol) was added to a solution of nicotinic acid (3.00 g, 24.4 mmol) in water (200 ml) to give a white precipitate. The mixture was stirred and treated dropwise with ethylene sulfide (1.45 ml, 24.4 mmol). The resulting yellow mixture was stirred at room temperature for 65 hours, then filtered to remove the solid portion which was washed with water (~15 ml), ethanol (~20 ml) and diethyl ether, and dried under vacuum to provide the intermediate silver mercaptide (4.60 g).

A portion (2.14 g) of the silver salt was suspended in water (25 ml) and the mixture was stirred rapidly with ice-bath cooling while hydrogen sulfide was bubbled in over 5 minutes. After stirring an additional 10 minutes at 0°, the black mixture was filtered through a celite® pad to remove the silver sulfide. The colorless filtrate was concentrated under vacuum to ca. 9 ml and added to a column of Dowex® 50W-X4 resin (hydrogen form) which was eluted with water, 1N hydrochloric acid, and 6N hydrochloric acid in a cold room. The product containing fractions were located in the 6N HCl eluant by UV, combined, and evaporated under vacuum to an oil containing crystals. This material was triturated with acetone to provide the title compound (153 mg) as a white solid.

NMR ($D_2C$) δ 3.04 (t, J = 6.4 Hz, $SCH_2$), 4.72 (t, J = 6.4 Hz, $NCH_2$), 8.88 (m, ArH), 8.91 (m, 2ArH), 9.28 (s, ArH).

EXAMPLE 3

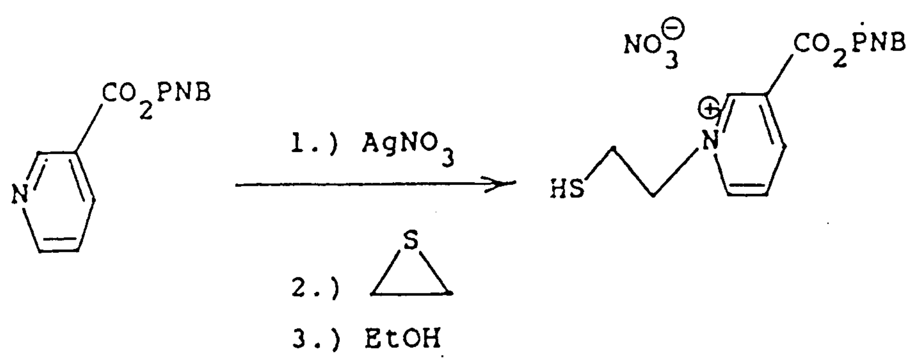


1-(2-Mercaptoethyl)-3-(p-nitrobenzyloxycarbonyl) pyridinium nitrate

A solution of silver nitrate (1.39 g, 8.19 mmol) in acetonitcile (10 ml) was added to a solution of p-nitrobenzyl nicotinate (2.00 g, 8.19 mmol) in acetonitrile (50 ml). A tan precipitate formed. The mixture was stirred at room temperature and treated with ethylene sulfide (0.49 ml, 8.19 mmol). After 1.5 hours, the mixture was filtered to remove the precipitate which dried under vacuum to an off-white solid. This material was suspended in ethanol (75 ml), treated with hydrogen sulfide over 5 minutes, and stirred an additional 5 minutes at room temperature. The mixture was centrifuged and filtered to remove the silver sulfide. The

filtrate, on standing overnight at room temperature, deposited the title compound (0.30 g) as fine white needles.
NMR (DMSO-$d_6$) δ 2.72 (t, J = 7 Hz, SH), 3.15 ( q, J = 7 Hz, $SCH_2$), 4.90 (t, J = 7 Hz, $NCH_2$), 5.66 (s, $CH_2Ar$), 7.87 (d, J = 8 Hz, 2 ArH), 8.33 (d, J = 8 Hz, ArH), 8.38 (m, ArH), 9.14 (d, J = 8 Hz, ArH), 9.31 (d, J = 6 Hz, ArH), 9.70 (s, ArH).

EXAMPLE 4

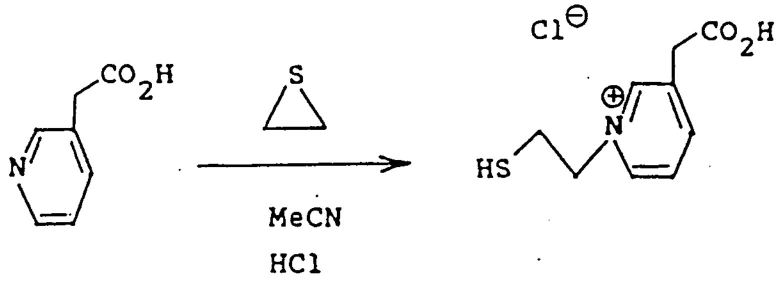


3-Carboxymethyl-1-(2-mercaptoethyl)pyridinium chloride

A mixture of 3-pyridylacetic acid (1.00 g, 7.3 mmol), 3-pyridylacetic acid hydrochloride (0.63 g, 3.65 mmol), ethylene sulfide (0.44 ml, 7.4 mmol) and acetonitrile (14 ml) was heated at reflux overnight. After cooling to room temperature, the lower, oily phase of the biphasic reaction mixture was separated and lyophilized from water (10 ml) to give the title compound (0.44 g) as an oil.
NMR ($D_2O$) δ 3.18 (m, $SCH_2$), 3.96 (s, $CH_2CO_2$), 4.80 (m, $NCH_2$), 8.08 (m, ArH), 8.54 (m, ArH), 8.84 (m, 2 ArH).

EXAMPLE 5

Step A:

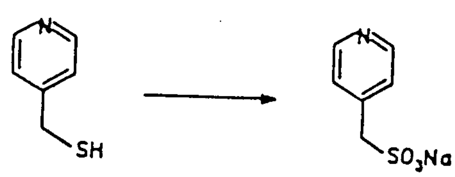

Sodium 4-pyridinemethanesulfonate

A solution of 4-mercaptomethylpyridine (2.20 g, 17.6 mmol) in acetonitrile (8.0 ml) was added to a stirred solution of 40% peracetic acid-acetic acid (25 ml) at ice bath temperature. After addition the solution was removed from the ice bath and stirred at room tenperature overnight. The solution was concentrated under vacuum to a white solid which was dissolved in water (1.5 ml) and adjusted to pH 9 with 2.5N aqueous sodium hydroxide. The resulting white suspension was filtered and the filtrate evaporated under vacuum to afford the title compound as a white solid (2.32 g).
NMR ($D_2O$) δ 4.24 (s, $CH_2$), 7.50 (d, J-5.4 Hz, ArH), 8.53 (d, J = 5.4 Hz, ArH).

STEP B:

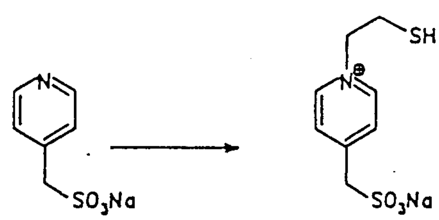

[1-(2-Mercaptoethyl)-4-pyridinio]methylsulfonate

A solution of silver nitrate (1.31 g, 7.68 mmol) in water (4 ml) was added to a stirred solution of sodium 4-pyridylmethylsulfonate (1.50 g, 7.68 mmol) in water (15 ml). The resulting hazy solution was treated with ethylene sulfide (0.51 ml, 8.58 mmol) added dropwise over 2 minutes. The resulting gummy mixture was let stand at room temperature 30 minutes and the supernatant decanted. The residue was mixed with water (10 ml) and vigorously bubbled with hydrogen sulfide for 10 minutes. The mixture was vigorously stirred an additional 1 hour and the silver sulfide precipitate removed by centrifugation. The supernatant was concentrated under vacuum to ca. 5 ml and charged onto a column of Dowex® 50W-X4 (hydrogen form, 0.037 - 0.074 mm (200-400 mesh), 1.5 x 33 cm). The column was eluted with water at 6.0 ml fractions/2.0 minutes. Fractions 19 to 27 combined and evaporated under vacuum to an off-white solid. The solid was mixed with a few mls of methanol and filtered to afford the title compound as an off-white solid (303 mg).
NMR ($D_2O$) δ 3.16 (t, J = 6.4 Hz, $SCH_2$), 4.52 (s, $CH_2SO_3^-$), 4.79 (t, J = 6.4 Hz, $NCH_2$), 8.12 (d, J = 6.2 Hz, ArH), 8.86 (d, J = 6.2 Hz, ArH).

| Anal. Calc'd for $C_8H_{11}NO_3S_2$: | | | | |
|---|---|---|---|---|
| | C, 41,18; | H, 4.75; | N, 6.00; | S, 27.48 |
| Found: | C, 41.32; | H, 4.77; | N, 6.07; | S, 27.17. |

STEP C:

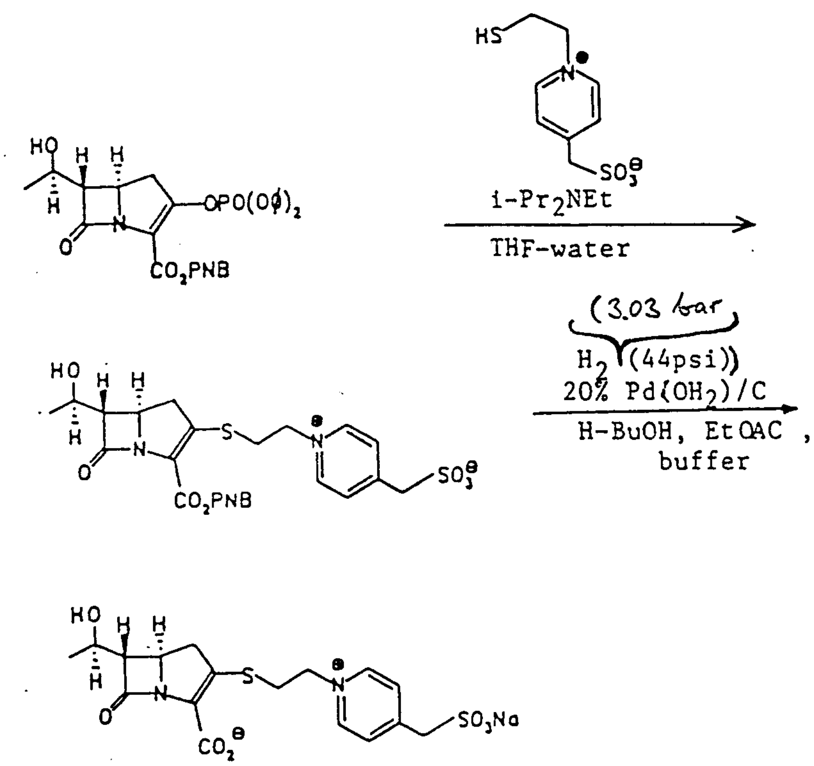


Sodium (5R, 6S)-6-[hydroxyethyl]-2-(2-(4-(2-sulfonatomethyl)pyridiniun)ethyl)thio-carbapen-2-em-3-carboxylate

A solution of p-nitrobenzyl (5R,6S)-2-(diphenylphosphono)oxy-6-[(IR)-hydroxyethyl]-carbapen-2-em-3-carpoxylate (300 mg, 0.517 mmol) and [1-(2-mercaptoethyl)-4-pyridinio]methylsulfonate (121mg, 0.517mmol) in tetrahydrofuran (2.0ml) and water (0.65ml) was treated at ice bath temperature with N,N-diisopropylethylamine (0.090ml, 0.517mmol). After stirring 10 min, the reaction solution was diluted with n-butanol (10.0ml), ethyl acetate (5.0ml), water (10.0ml), and 0.5M pH 6.8 N-methylmorpholine - hydrochloric acid buffer (5.0 ml), mixed with 10% palladium on carbon (125 mg), and hydrogenated on a Parr® shaker at 3.10 bar (45 psi) for 75 min. The catalyst was removed by filtration through a prewashed celite® pad, and the filtrate washed with methylene chloride. The aqueous phase was concentrated under vacuum to ca. 7.5 ml and charged onto a column of Dowex® 50W-X4 (sodium form, 0.037 - 0.074 mm (200-400 mesh), 1.5 x 33 cm). The column was eluted with water in the cold room (4°) at 6.0 ml fractions;2.0 min. Fractions 4 to 8 were combined and concentrated under vacuum to ca. 8ml and lyophilized to powder. This material was chromatographed on a 5 1.0mm 20x20cm Analtech RPS-F plate using water as a developing solvent in a cold room (4°). The major uv visible band on each plate at Rf 0.3-0.5 was removed and eluted with 4:1 acetonitrile-water. The eluant was washed with hexane, concentrated under vacuum to ca. 20 ml, filtered through an Acrodisc (Gelman, 0.2 micron CR) and lyophilized to afford the title compound (100.3mg) as an amorphous white powder.

IR (Nujol) $\nu$ max 3400 (br), 1740, 1640, 1585 $cm^{-1}$

uv (water) $\lambda$ max $\lambda$ max 296 ($\epsilon$ 7,230) 260nm ($\epsilon$ 6320), 229nm ($\epsilon$ 11,400) (water + $NH_2OH$. HCl + $K_2HPO_4$) extinguished $\lambda$ max 297nm ($\epsilon$ 6,510).

NMR (D$_2$O) δ 1.26 (d, J = 6.4 Hz, CH$_3$), 2.94 (dd, J = 8.9, 17.2 Hz, CH$_a$H$_b$), 3.08 (dd, J = 9.5, 172 Hz, CH$_A$H$_B$), 3.34 (m, SCH$_A$H$_B$), 3.35 (m, H$_6$), 3.53 (td, J = 6.0, 15.2 Hz, SCH$_A$H$_B$), 4.07 (dt, J = 2.5, 9.5Hz, H5), 4.20 (~ p, J = 6.4 Hz, CH$_3$CHOH), 4.52 (s, CH$_2$SO$_3^{\ominus}$ 4.9 (M, NCH$_2$), 8.11 (d, J = 6.4Hz, ArH) 8.85 (d, J = 6.4Hz, ArH).

EXAMPLE 6

Step A

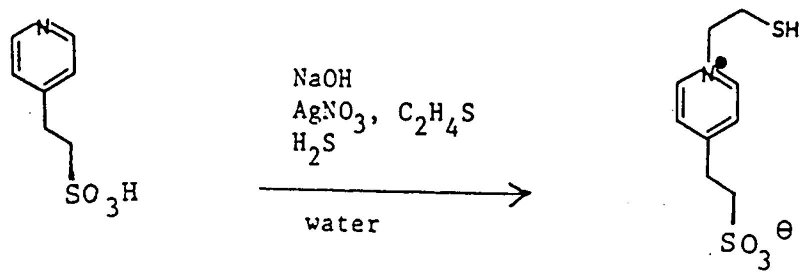


[1-(2-Mercaptoethyl)-4-pyridinio]-2-ethylsulfonate

A solution of silver nitrate (1.84 g, 10.6 mmol) in water (5 ml) was added to a stirred aqueous solution (20 ml) of 4-pyridine ethane sulfonic acid (2.00 g, 10.6 mmol) which had been adjusted to pH 7 by the addition of aqueous 2.5N sodium hydroxide (3.0 ml). The solution was cooled in an ice-water bath and ethylene sulfide (0.71 ml, 11.8 mmol) added. The resulting mixture was let stand 45 minutes at ice-bath temperature and the upper clear phase decanted. The gummy solid was mixed with water (20 ml) and vigorously bubbled with hydrogen sulfide for 15 minutes. After stirring an additional 20 minutes, the mixture was filtered through a celite® pad to remove silver sulfide and the filtrate was evaporated under vacuum to provide crude title compound as a white semi-solid (1.00 g).

STEP B:

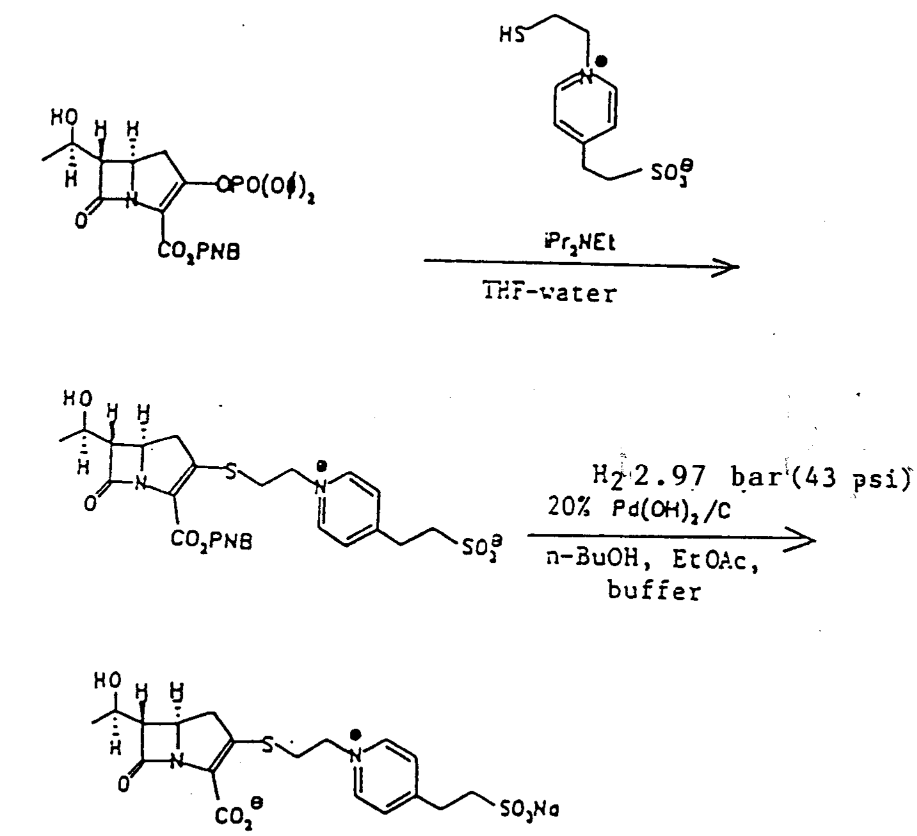


Sodium (5R,6S)-6-[(hydroxyethyl]-2-(2-(4-(2-sulfonatoethyl)-1-pyridinium)ethyl)thio-carbapen-2-em-3-carboxylate

A solution of p-nitrobenzyl (5R,6S)-2-(diphenylphosphono)oxy-6-[(1R)-hydroxyethyl]-carbapen-2-em-3-carboxylate (200 mg, 0.344 mmol) and [1-(2-mercaptoethyl)-4-pyridinio]-2-ethylsulfonate (89 mg, 0.362 mmol) in tetrahydrofuran (1.0 ml) and water (0.3 ml) was treated at room temperature with N,N-diisopropylethylamine (0.063 ml, 0.362 mmol). After stirring 10 minutes, the reaction solution was diluted with n-butanol (6.8 ml), ethyl acetate (3.4 ml), water (6.8 ml), and 0.5M pH 6.8 N-methylmorpholine-hydrochloric acid buffer (3.4 ml), mixed with 20% palladium hydroxide on carbon (80 mg), and hydrogenated on a Parr® shaker at 3.03 bar (44 psi) for 75 minutes. The catalyst was removed by filtration through an Acrodisc (Gelman, 0.45 micron CR), and the filtrate washed with methylene chloride. The aqueous phase was concentrated under vacuum to ca. 8.5 ml and charged onto a column of Dowex® 50W-X4 (sodium form, 0.037 - 0.074 mm (200-400 mesh), 1.5 x 35 cm). The column was eluted with water in the cold room (4°) at 4.0 ml fractions/1.5 minutes. Fractions 6 to 13 were combined and concentrated under vacuum to ca. 9 ml and lyophilized to powder. This material was chromatographed on three 1.0 mm 20 x 20 cm Analtech RPS-F plates using 2% ethanol-water as a developing solvent in a cold room (4°). The major UV visible band on each plate at Rf 0.4-0.6 was removed and eluted with 4:1 acetonitrile-water. The eluant was washed with hexane, concentrated under vacuum to ca. 7 ml, filtered through an Acrodisc (Gelman, 0.45 micron CR) and lyophilized to afford the title compound (45 mg) as an amorphous white powder.

| | |
|---|---|
| ir | (Nujol) 3350 (br), 1748, 1640, 1590 $cm^{-1}$ |
| UV | (water) λ max 297 nm (ε 7,020), λ max 256 nm (ε 5,500), λ max 224 nm (ε 10,500) |
| UV | (water + $NH_2OH.HCl$ + $K_2HPO_4$) extinguished λ max 297 nm (ε 6,580) |
| NMR | ($D_2O$) δ 1.28 (d, J=6.4 Hz, $CH_3CHOH$), 2.94 (dd, J=8.7, 17.5 Hz, $CH_aH_b$), 3.10 (dd, J=9.6, 17.5 Hz, $CH_aH_b$), 3.35 (dd, J=2.8, 6.0 Hz, H6), 3.3-3.4 (m, $CH_2CH_2SO_3$), 3.3-3.4 (m, $SCH_aH_b$), 3.53 (td, J=6.1, 15.0 Hz, $SCH_aH_b$), 4.05 (dt, J=2,7, 9.2 Hz, H5), 4.22 (~p, J=6 Hz, CHOH), |

8.02 (d, J=6.6 Hz, ArH), 8.76 (d, J=6.6 Hz, ArH).

EXAMPLE 7

Using procedures as described in Examples 1-6 the following formula I compounds are prepared:

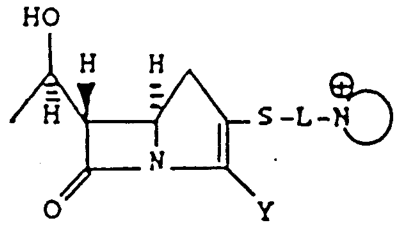

I

| Compound No. | L | 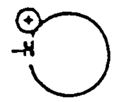 | Y |
|---|---|---|---|
| 1 | $-CH_2CH_2-$ | 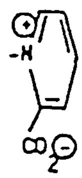 | $-CO_2Na$ |
| 2 | $-CH_2CH_2-$ | 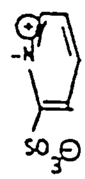 | $-CO_2Na$ |
| 3 | $-CH_2CH_2-$ | 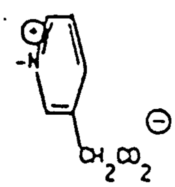 | $-CO_2Na$ |
| 4 | $-CH_2CH_2-$ | 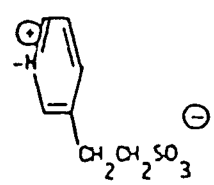 | $-CO_2Na$ |

| Compound No. | L | 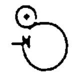 | Y |
|---|---|---|---|
| 5 | $-CH_2CH_2-$ | 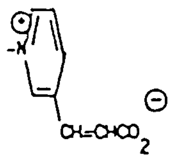 | $-CO_2Na$ |
| 6 | $-CH_2CH_2-$ | 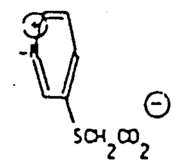 | $-CO_2Na$ |
| 7 | $-CH_2CH_2-$ | 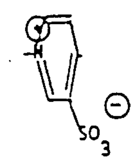 | $-CO_2Na$ |
| 8 | $-CH_2CH_2-$ | 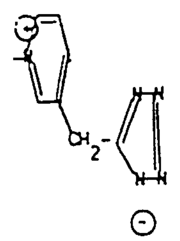 | $-CO_2Na$ |
| 9 | $-CH_2CH_2-$ | 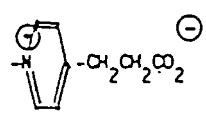 | $-CO_2Na$ |
| 10 | $-CH_2CH_2-$ | 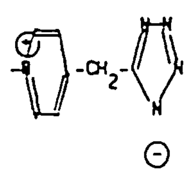 | $-CO_2Na$ |
| 11 | $-CH_2CH_2-$ | 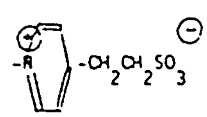 | $-CO_2Na$ |

| Compound No. | L | 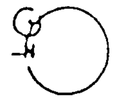 | Y |
|---|---|---|---|
| 12 | $-CH_2CH_2-$ | 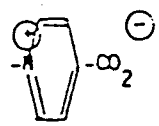 | $-CO_2Na$ |
| 13 | $-CH_2CH_2-$ | 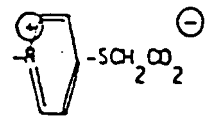 | $-CO_2Na$ |
| 14 | $-CH(CH_3)CH_2-$ | 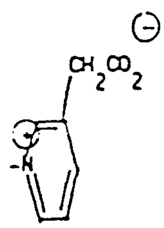 | $-CO_2Na$ |
| 15 | $-CH(CH_3)CH_2-$ | 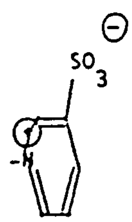 | $-CO_2Na$ |

| Compound No. | L | 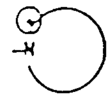 | Y |
|---|---|---|---|
| 16 | $-CH(CH_3)CH_2-$ | 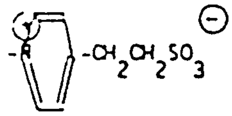 | $-CO_2Na$ |
| 17 | $-CH(CH_3)CH_2-$ | 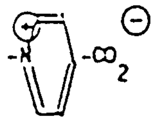 | $-CO_2Na$ |
| 18 | $-CH_2CH_2-$ | 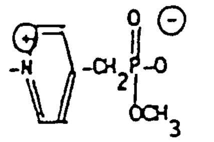 | $-CO_2Na$ |
| 19 | $-CH_2CH_2-$ | 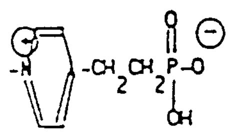 | $-CO_2Na$ |

| Compound No. | L | 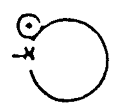 | Y |
|---|---|---|---|
| 20 | $-CH_2CH_2-$ | 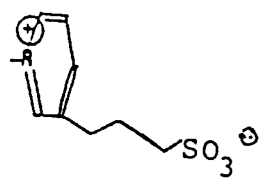 | $-CO_2Na$ |
| 21 | $-CH_2CH_2-$ | 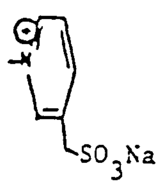 | $-CO_2^{\ominus}$ |
| 22 | $-CH_2CH_2-$ | 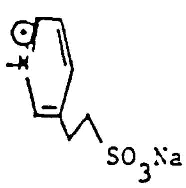 | $-CO_2^{\ominus}$ |

| Compound No. | L | 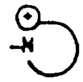 | Y |
|---|---|---|---|
| 23 | $-CH_2CH_2-$ | 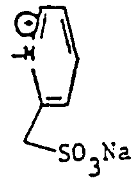 | $-CO_2^{\ominus}$ |
| 24 | $-CH_2CH_2-$ | 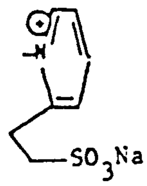 | $-CO_2^{\ominus}$ |

| Compound No. | L | 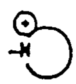 | Y |
|---|---|---|---|
| 25 | $-CH_2CH_2-$ | 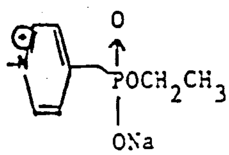 | $-CO_2^{\ominus}$ |
| 26 | $-CH_2CH_2-$ | 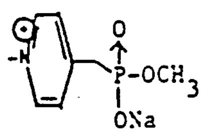 | $-CO_2^{\ominus}$ |
| 27 | $-CH_2CH_2-$ | 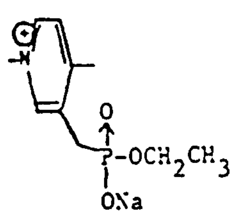 | $-CO_2^{\ominus}$ |

| Compound No. | L | 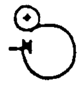 | Y |
| --- | --- | --- | --- |
| 28 | $-CH_2CH_2-$ | 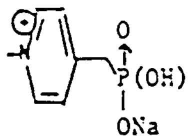 | $-CO_2^{\ominus}$ |
| 29 | $-CH_2CH_2-$ | 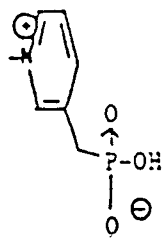 | $-CO_2^{\ominus}$ |

| Compound No. | L | 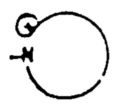 | Y |
| --- | --- | --- | --- |
| 30 | $-CH_2CH_2-$ | 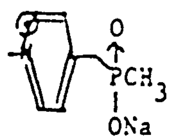 | $-CO_2^{\ominus}$ |
| 31 | $-CH_2CH_2-$ | 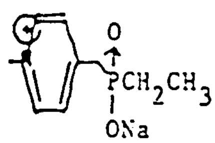 | $-CO_2^{\ominus}$ |

| Compound No. | L | 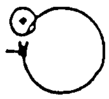 | Y |
|---|---|---|---|
| 32 | $-CH_2CH_2-$ | 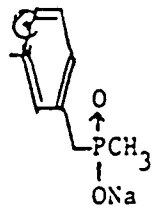 | $-CO_2^{\ominus}$ |
| 33 | $-CH_2CH_2-$ | 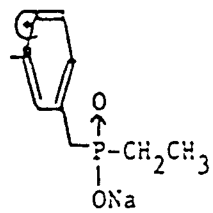 | $-CO_2^{\ominus}$ |
| 34 | $-CH_2CH_2-$ | 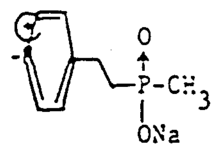 | $-CO_2^{\ominus}$ |
| 35 | $-CH_2CH_2-$ | 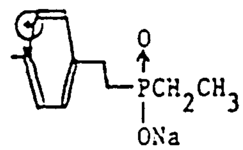 | $-CO_2^{\ominus}$ |
| 36 | $-CH_2CH_2-$ | 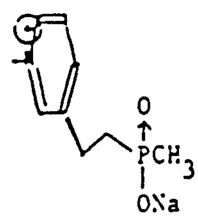 | $-CO_2^{\ominus}$ |
| 37 | $-CH_2CH_2-$ | 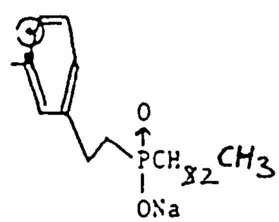 | $-CO_2^{\ominus}$ |

| Compound No. | L | 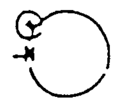 | Y |
|---|---|---|---|
| 38 | $-CH_2CH_2-$ | 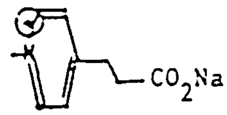 | $-CO_2^{\ominus}$ |

| Compound No. | L | 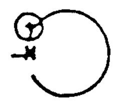 | Y |
|---|---|---|---|
| 39 | $-CH_2CH_2-$ | 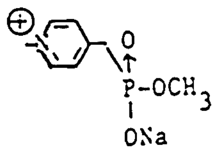 | $-CO_2^{\ominus}$ |
| 40 | $-CH_2CH_2-$ | 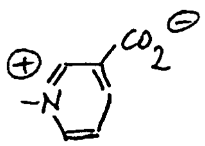 | $-CO_2Na$ |
| 41 | $-CH_2CH_2-$ | 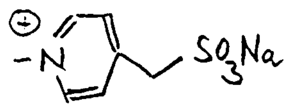 | $-CO_2^{\ominus}$ |

## Claims

**Claims for the following Contracting States : BE, CH, DE, FR, GB, IT, LI, LU, NL, SE**

**1.** A compound having the formula:

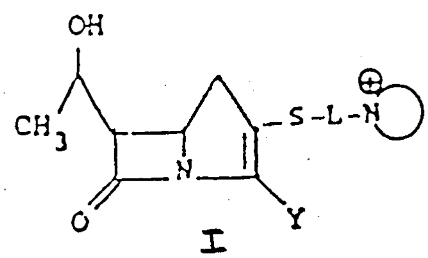

I

wherein 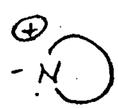 is 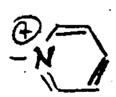

wherein the pyridinium group is required to be substituted by:

A: an acidic side-chain of the structure A or $-(CH_2)_n-X-(CH_2)_m-Y'-A$ where:

n = 0 - 4

m = 0 - 4

X = $CHR^s$, CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O;

$R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A = an acidic function selected from carboxy ($CO_2H$), phosphono [P=O(OH)$_2$], alkylphosphono {P=O(OH)-[O($C_1$-$C_4$ alkyl)]}, alkylphosphinyl [P=O(OH)-($C_1$-$C_4$ alkyl)], phosphoramide [P=O(OH)-NH($C_1$-$C_4$ alkyl) and P=O(OH)$NHR^x$], sulfino ($SO_2H$), sulfo ($SO_3H$), 5-tetrazolyl ($CN_4H$), heteroarylsulfonamido ($SO_2NHR^x$) and acylsulfonamides represented by the structures $CONHSO_2$ ($C_1$-$C_4$ alkyl), $CONHSO_2N$($C_1$-$C_4$ alkyl)$_2$, $SO_2NHCO$($C_1$-$C_4$ alkyl) and $SO_2NHCOR^x$;

$R^x$ = monocyclic or bicyclic heteroaryl group containing (a) when monocyclic, up to 3 heteroatoms and up to 6 total ring atoms or (b) when bicyclic up to 5 heteroatoms and 9-10 ring atoms in which the heteroatoms are selected from nitrogen, oxygen and sulfur;

and optionally by

B: a halogen atom;

L is a straight or branched chain, bivalent $C_1$-$C_6$ alkylene, $C_2$-$C_6$ alkenylene or $C_3$-$C_6$ cycloalkylene, or -$C_1$-$C_4$ alkylene-X-$C_1$-$C_4$ alkylene wherein X is O, S, NH, or N($C_1$-$C_4$ alkyl);

and

Y is

i) -COOH, a pharmaceutically acceptable ester or salt thereof;

ii) COOR wherein R is a removable carboxy protecting group;

iii) COOM wherein M is an alkali metal; or

iv) $COO^-$ provided that when Y is other than iv) a counterion $Z^\ominus$ is present.

**2.** A compound of claim 1 wherein the compound is a member selected from the group consisting of:

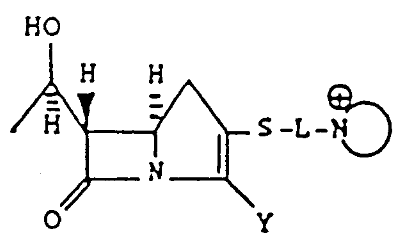

I

| Compound No. | L | 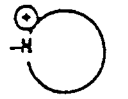 | Y |
|---|---|---|---|
| 1 | $-CH_2CH_2-$ | 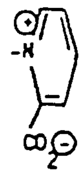 | $-CO_2Na$ |
| 2 | $-CH_2CH_2-$ | 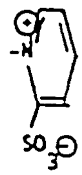 | $-CO_2Na$ |
| 3 | $-CH_2CH_2-$ | 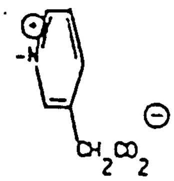 | $-CO_2Na$ |
| 4 | $-CH_2CH_2-$ | 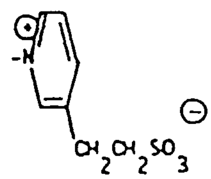 | $-CO_2Na$ |

| Compound No. | L | 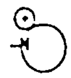 | Y |
|---|---|---|---|
| 5 | $-CH_2CH_2-$ | 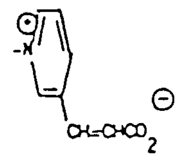 | $-CO_2Na$ |
| 6 | $-CH_2CH_2-$ | 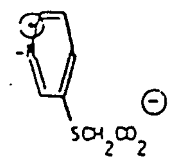 | $-CO_2Na$ |
| 7 | $-CH_2CH_2-$ | 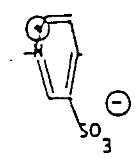 | $-CO_2Na$ |
| 8 | $-CH_2CH_2-$ | 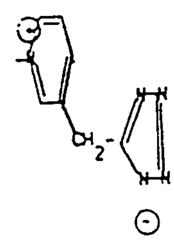 | $-CO_2Na$ |
| 9 | $-CH_2CH_2-$ | 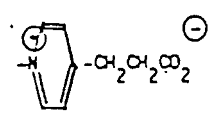 | $-CO_2Na$ |
| 10 | $-CH_2CH_2-$ | 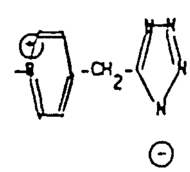 | $-CO_2Na$ |
| 11 | $-CH_2CH_2-$ | 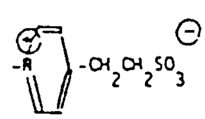 | $-CO_2Na$ |

| Compound No. | L | 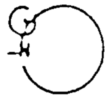 | Y |
|---|---|---|---|
| 12 | $-CH_2CH_2-$ | 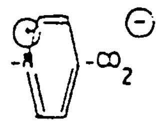 | $-CO_2Na$ |
| 13 | $-CH_2CH_2-$ | 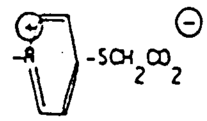 | $-CO_2Na$ |
| 14 | $-CH(CH_3)CH_2-$ | 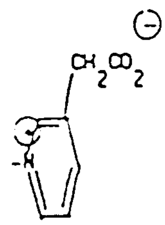 | $-CO_2Na$ |
| 15 | $-CH(CH_3)CH_2-$ | 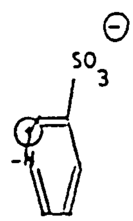 | $-CO_2Na$ |

| Compound No. | L | 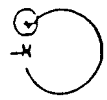 | Y |
|---|---|---|---|
| 16 | $-CH(CH_3)CH_2-$ | 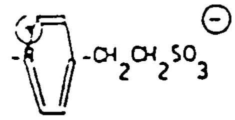 | $-CO_2Na$ |
| 17 | $-CH(CH_3)CH_2-$ | 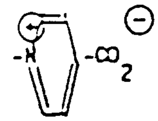 | $-CO_2Na$ |
| 18 | $-CH_2CH_2-$ | 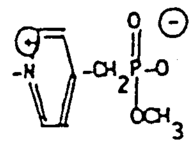 | $-CO_2Na$ |
| 19 | $-CH_2CH_2-$ | 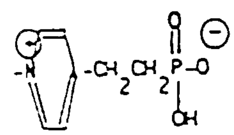 | $-CO_2Na$ |

| Compound No. | L | 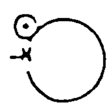 | Y |
|---|---|---|---|
| 20 | $-CH_2CH_2-$ | 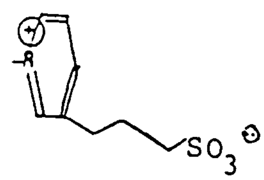 | $-CO_2Na$ |
| 21 | $-CH_2CH_2-$ | 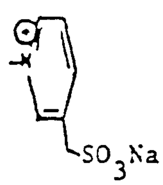 | $-CO_2^{\ominus}$ |
| 22 | $-CH_2CH_2-$ | 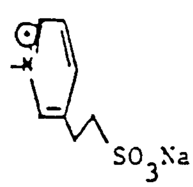 | $-CO_2^{\ominus}$ |

| Compound No. | L | 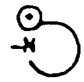 | Y |
|---|---|---|---|
| 23 | $-CH_2CH_2-$ | 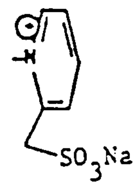 | $-CO_2^{\ominus}$ |
| 24 | $-CH_2CH_2-$ | 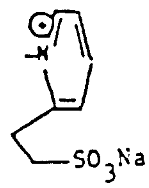 | $-CO_2^{\ominus}$ |

| Compound No. | L | 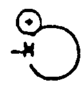 | Y |
|---|---|---|---|
| 25 | $-CH_2CH_2-$ | 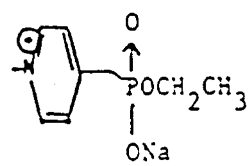 | $-CO_2^{\ominus}$ |
| 26 | $-CH_2CH_2-$ | 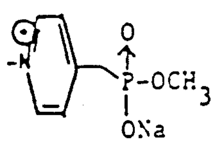 | $-CO_2^{\ominus}$ |
| 27 | $-CH_2CH_2-$ | 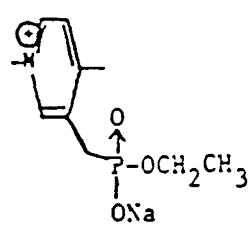 | $-CO_2^{\ominus}$ |

| Compound No. | L | 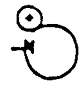 | Y |
|---|---|---|---|
| 28 | $-CH_2CH_2-$ | 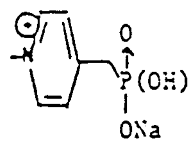 | $-CO_2^{\ominus}$ |
| 29 | $-CH_2CH_2-$ | 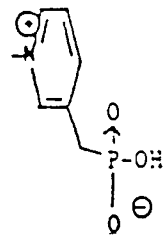 | $-CO_2^{\ominus}$ |

| Compound No. | L | 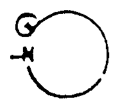 | Y |
|---|---|---|---|
| 30 | $-CH_2CH_2-$ | 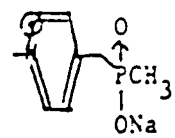 | $-CO_2^{\ominus}$ |
| 31 | $-CH_2CH_2-$ | 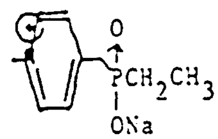 | $-CO_2^{\ominus}$ |

| Compound No. | L | 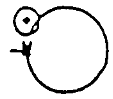 | Y |
|---|---|---|---|
| 32 | $-CH_2CH_2-$ | 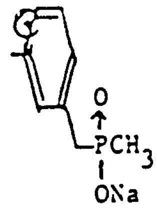 | $-CO_2^{\ominus}$ |
| 33 | $-CH_2CH_2-$ | 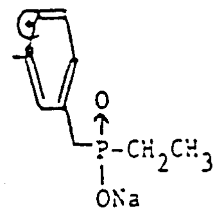 | $-CO_2^{\ominus}$ |
| 34 | $-CH_2CH_2-$ | 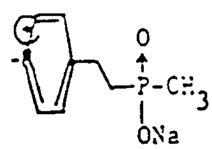 | $-CO_2^{\ominus}$ |
| 35 | $-CH_2CH_2-$ | 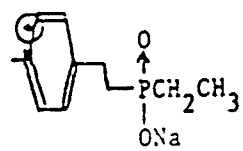 | $-CO_2^{\ominus}$ |
| 36 | $-CH_2CH_2-$ | 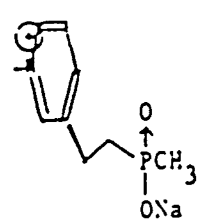 | $-CO_2^{\ominus}$ |
| 37 | $-CH_2CH_2-$ | 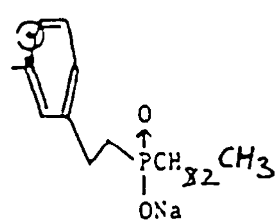 | $-CO_2^{\ominus}$ |

| Compound No. | L | 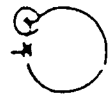 | Y' |
|---|---|---|---|
| 38 | $-CH_2CH_2-$ | 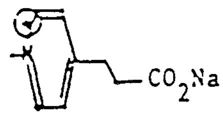 | $-CO_2^{\ominus}$ |

| Compound No. | L | 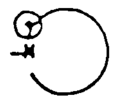 | Y' |
|---|---|---|---|
| 39 | $-CH_2CH_2-$ | 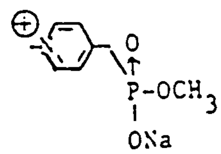 | $-CO_2^{\ominus}$ |
| 40 | $-CH_2CH_2-$ | 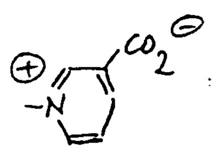 | $-CO_2Na$ |
| 41 | $-CH_2CH_2-$ | 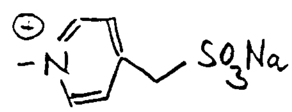 | $-CO_2^{\ominus}$ |

**3.** A compound of claim 1 of the structure:

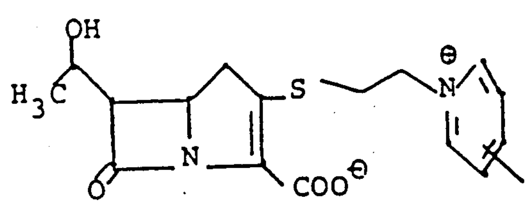

A

wherein

A is an acidic side-chain of the structure $-(CH_2)_n-X-(CH_2)_m-Y'-A$ as defined in claim 1.

**4.** A compound having the formula:

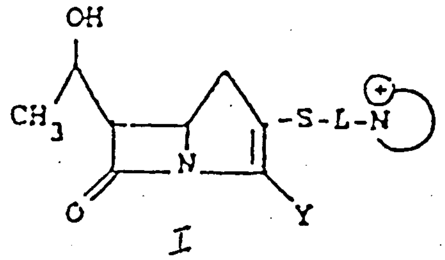

I

wherein L and Y are as defined in claim 1 and

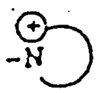 is 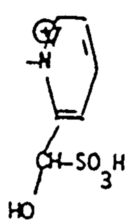, 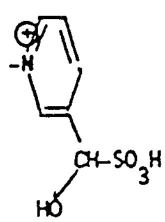, 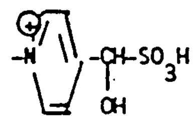,

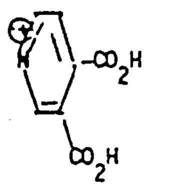 or 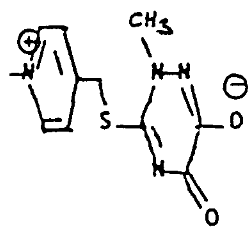.

**5.** A compound of claim 4 having the formula:

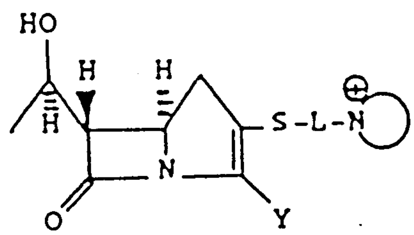

I

wherein L is $-CH_2CH_2-$, Y is $-CO_2Na$ and

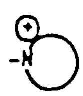

is

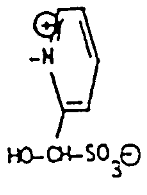, 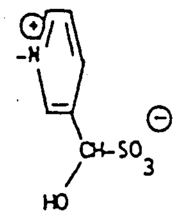, 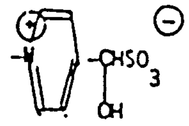 or 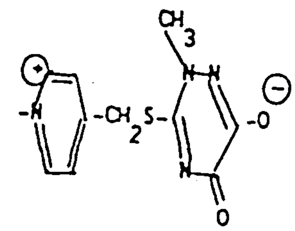 .

6. A combination of a compound according to any of claims 1 to 5 and a DHP inhibitor.

7. A combination of claim 6 wherein the DHP inhibitor is 7-(L-2-amino-2-carboxyethylthio)-2-(2,2-dimethylcyclopropanecarboxamide)-2-heptenoic adid.

8. A pharmaceutical composition for antibiotic use comprising an antibacterially effective amount of a compound of any one of claims 1 to 5 an inhibitorily effective amount of a DHP inhibitor, and, optionally, a pharmaceutically acceptable carrier.

**Claims for the following Contracting State : AT**

1. A process for preparing a compound having the formula:

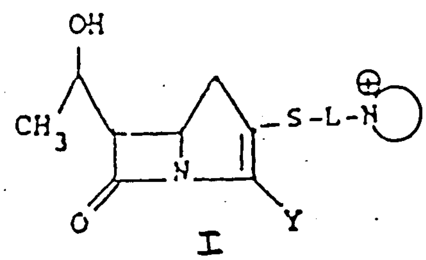

I

wherein 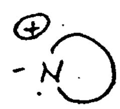 is 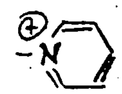 and

wherein the pyridinium group is required to be substituted by:

A: an acidic side-chain of the structure A or $-(CH_2)_n-X-(CH_2)_m-Y'-A$ where:

n = 0 - 4

m = 0 - 4

X = $CHR^s$, CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O;

$R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A = an acidic function selected from carboxy ($CO_2H$), phosphono [P=O(OH)$_2$], alkylphosphono {P=O(OH)-[O($C_1$-$C_4$ alkyl)]}, alkylphosphinyl [P=O(OH)-($C_1$-$C_4$ alkyl)], phosphoramido [P=O(OH)-NH($C_1$-$C_4$ alkyl) and P=O(OH)NH$R^x$], sulfino ($SO_2H$), sulfo ($SO_3H$), 5-tetrazolyl ($CN_4H$), heteroarylsulfonamido ($SO_2NHR^x$) and acylsulfonamides represented by the structures $CONHSO_2$($C_1$-$C_4$ alkyl), $CONHSO_2N$($C_1$-$C_4$ alkyl)$_2$, $SO_2NHCO$($C_1$-$C_4$ alkyl) and $SO_2NHCOR^x$;

$R^x$ = monocyclic or bicyclic heteroaryl group containing (a) when monocyclic, up to 3 heteroatoms and up to 6 total ring atoms or (b) when bicyclic up to 5 heteroatoms and 9-10 ring atoms in which the heteroatoms are selected from nitrogen, oxygen and sulfur;

and optionally by

B: a halogen atom;

L is a straight or branched chain, bivalent $C_1$-$C_6$ alkylene, $C_2$-$C_6$ alkenylene or $C_3$-$C_6$ cycloalkylene, or -$C_1$-$C_4$ alkylene-X-$C_1$-$C_4$ alkylene wherein X is O, S, NH, or N($C_1$-$C_4$ alkyl);

and

Y is

i) -COOH, a pharmaceutically acceptable ester or salt thereof;
ii) COOR wherein R is a removable carboxy protecting group;
iii) COOM wherein M is an alkali metal; or
iv) $COO^-$ provided that when Y is other than iv) a counterion $Z^{\ominus}$ is present;

which comprises:

A. reaction of a compound of formula

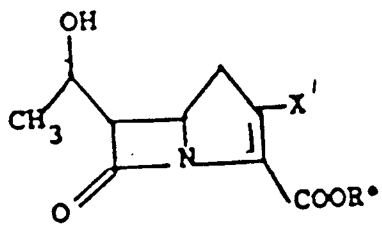

with a compound of formula

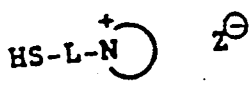

in the presence of a base and possibly deblocking the resulting compound of formula

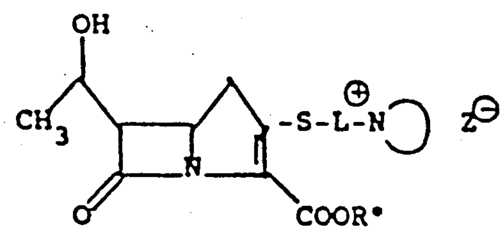

to obtain

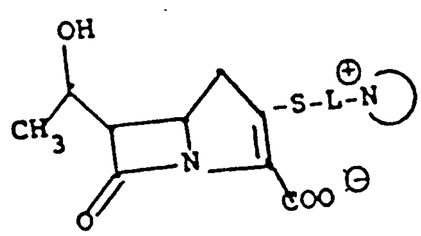

where R° is other than (-) or H and is a readily removable blocking group and X' is a leaving group; or

B. reacting a compound of formula

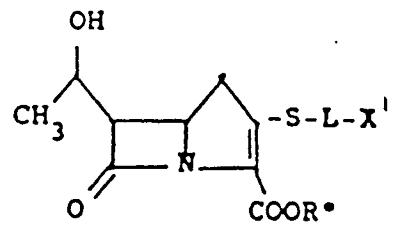

where X' is a leaving group and R° is as defined above, with

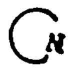

in the presence of a base or $Ag^{\oplus}$ and optionally deblocking the resulting compound of formula

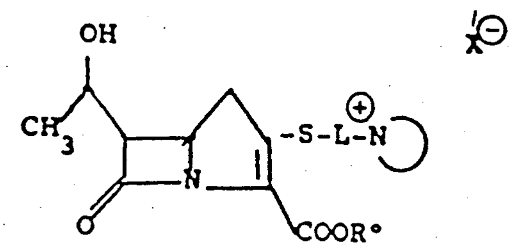

to obtain

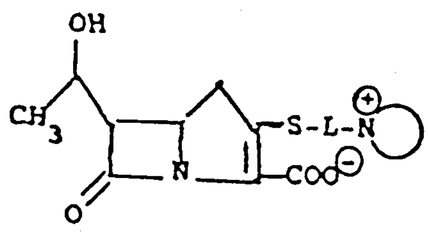

or C.reacting the compound of formula

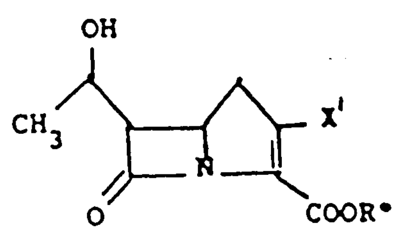

where R°, X' and Z are as previously defined and X'' is a leaving group with NaHS, reacting with the resulting compound of formula

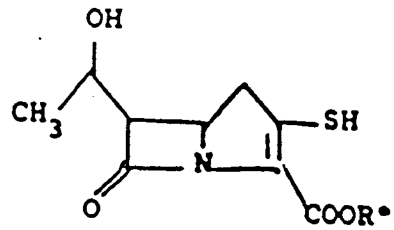

with

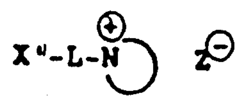

and optionally deblocking the resulting compound of formula

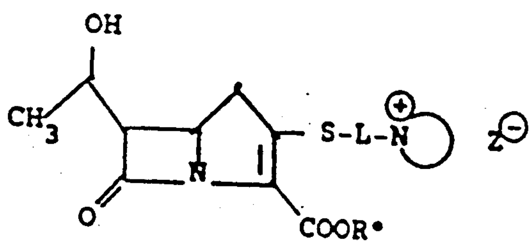

to obtain

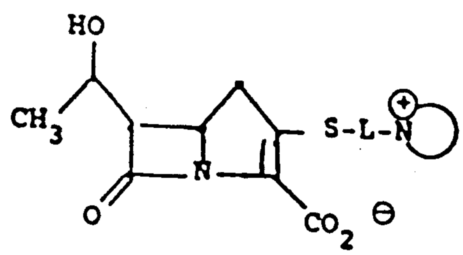

**2.** A process of claim 1 for preparing a compound which is a member selected from the group consisting of:

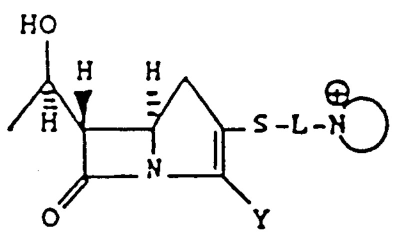

I

| Compound No. | L | 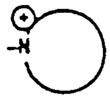 | Y |
|---|---|---|---|
| 1 | $-CH_2CH_2-$ | 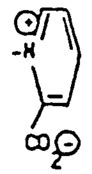 | $-CO_2Na$ |
| 2 | $-CH_2CH_2-$ | 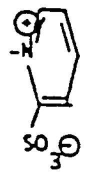 | $-CO_2Na$ |
| 3 | $-CH_2CH_2-$ | 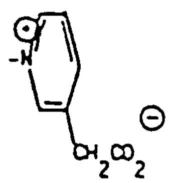 | $-CO_2Na$ |
| 4 | $-CH_2CH_2-$ | 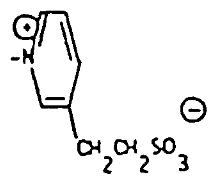 | $-CO_2Na$ |

| Compound No. | L | 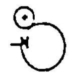 | Y |
|---|---|---|---|
| 5 | $-CH_2CH_2-$ | 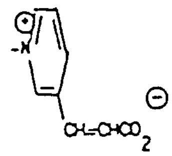 | $-CO_2Na$ |
| 6 | $-CH_2CH_2-$ | 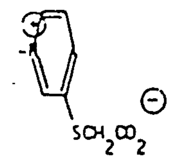 | $-CO_2Na$ |
| 7 | $-CH_2CH_2-$ | 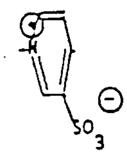 | $-CO_2Na$ |
| 8 | $-CH_2CH_2-$ | 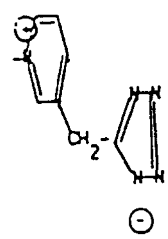 | $-CO_2Na$ |
| 9 | $-CH_2CH_2-$ | 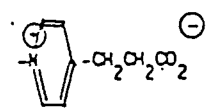 | $-CO_2Na$ |
| 10 | $-CH_2CH_2-$ | 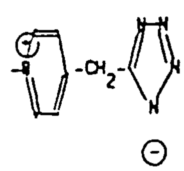 | $-CO_2Na$ |
| 11 | $-CH_2CH_2-$ | 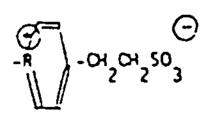 | $-CO_2Na$ |

| Compound No. | L | 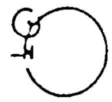 | Y |
|---|---|---|---|
| 12 | $-CH_2CH_2-$ | 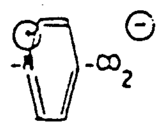 | $-CO_2Na$ |
| 13 | $-CH_2CH_2-$ | 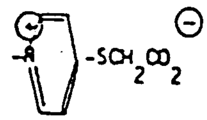 | $-CO_2Na$ |
| 14 | $-CH(CH_3)CH_2-$ | 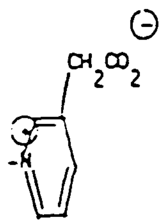 | $-CO_2Na$ |
| 15 | $-CH(CH_3)CH_2-$ | 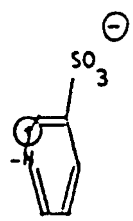 | $-CO_2Na$ |

| Compound No. | L | 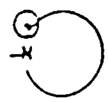 | Y |
|---|---|---|---|
| 16 | $-CH(CH_3)CH_2-$ | 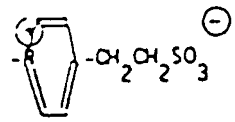 | $-CO_2Na$ |
| 17 | $-CH(CH_3)CH_2-$ | 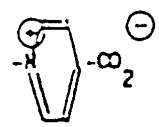 | $-CO_2Na$ |
| 18 | $-CH_2CH_2-$ | 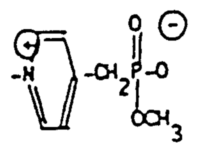 | $-CO_2Na$ |
| 19 | $-CH_2CH_2-$ | 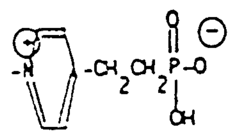 | $-CO_2Na$ |

| Compound No. | L | 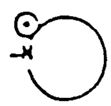 | Y |
|---|---|---|---|
| 20 | $-CH_2CH_2-$ | 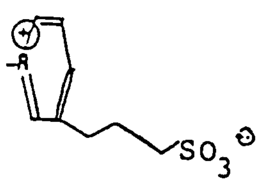 | $-CO_2Na$ |
| 21 | $-CH_2CH_2-$ | 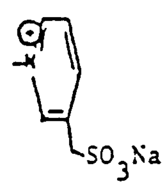 | $-CO_2^{\ominus}$ |
| 22 | $-CH_2CH_2-$ | 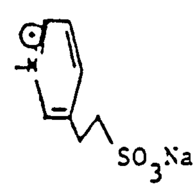 | $-CO_2^{\ominus}$ |

| Compound No. | L | 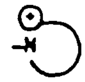 | Y |
|---|---|---|---|
| 23 | $-CH_2CH_2-$ | 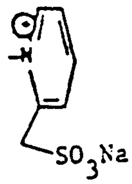 | $-CO_2^{\ominus}$ |
| 24 | $-CH_2CH_2-$ | 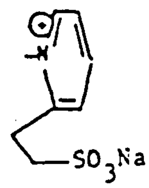 | $-CO_2^{\ominus}$ |

| Compound No. | L | 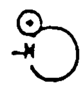 | Y |
|---|---|---|---|
| 25 | $-CH_2CH_2-$ | 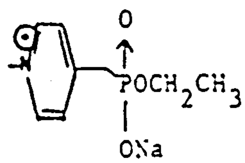 | $-CO_2^{\ominus}$ |
| 26 | $-CH_2CH_2-$ | 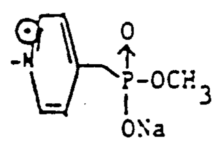 | $-CO_2^{\ominus}$ |
| 27 | $-CH_2CH_2-$ | 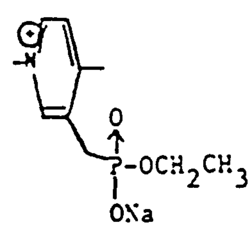 | $-CO_2^{\ominus}$ |

| Compound No. | L | 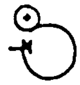 | Y |
|---|---|---|---|
| 28 | $-CH_2CH_2-$ | 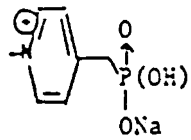 | $-CO_2^{\ominus}$ |
| 29 | $-CH_2CH_2-$ | 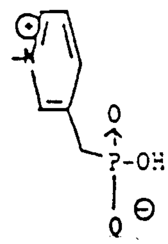 | $-CO_2^{\ominus}$ |

| Compound No. | L | 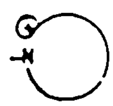 | Y |
|---|---|---|---|
| 30 | $-CH_2CH_2-$ | 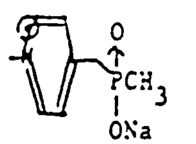 | $-CO_2^{\ominus}$ |
| 31 | $-CH_2CH_2-$ | 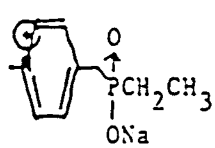 | $-CO_2^{\ominus}$ |

| Compound No. | L | 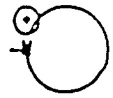 | Y |
|---|---|---|---|
| 32 | $-CH_2CH_2-$ | 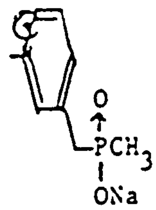 | $-CO_2^{\ominus}$ |
| 33 | $-CH_2CH_2-$ | 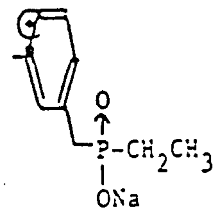 | $-CO_2^{\ominus}$ |
| 34 | $-CH_2CH_2-$ | 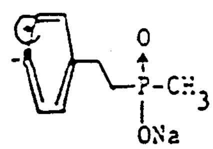 | $-CO_2^{\ominus}$ |
| 35 | $-CH_2CH_2-$ | 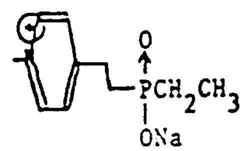 | $-CO_2^{\ominus}$ |
| 36 | $-CH_2CH_2-$ | 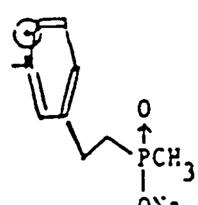 | $-CO_2^{\ominus}$ |
| 37 | $-CH_2CH_2-$ | 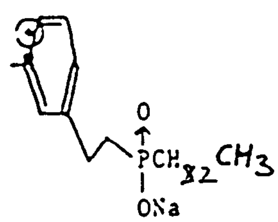 | $-CO_2^{\ominus}$ |

| Compound No. | L | 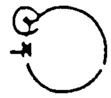 | Y |
|---|---|---|---|
| 38 | $-CH_2CH_2-$ | 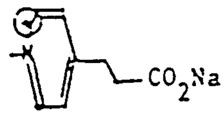 | $-CO_2^{\ominus}$ |

| Compound No. | L | 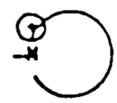 | Y |
|---|---|---|---|
| 39 | $-CH_2CH_2-$ | 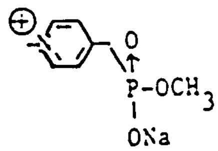 | $-CO_2^{\ominus}$ |
| 40 | $-CH_2CH_2-$ | 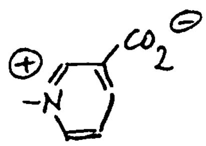 | $-CO_2Na$ |
| 41 | $-CH_2CH_2-$ | 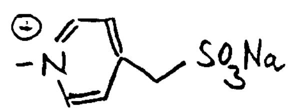 | $-CO_2^{\ominus}$ |

3. A process of claim 1 for preparing a compound of the structure:

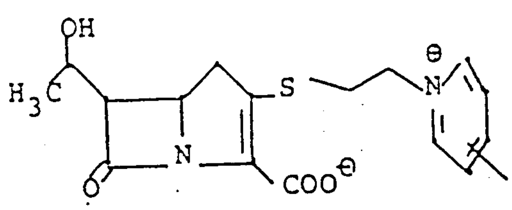

A

wherein

A is an acidic side-chain of the structure $-(CH_2)_n-X-(CH_2)_m-Y'-A$ as defined in claim 1.

**4.** A process for preparing a compound having the formula:

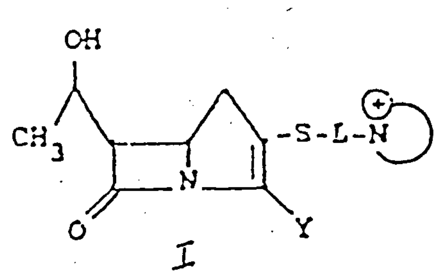

I

wherein L and Y are as defined in claim 1 and

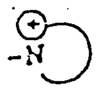 is 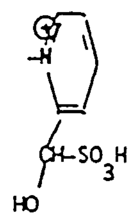 , 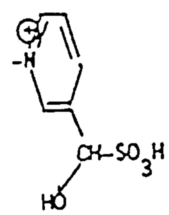 , 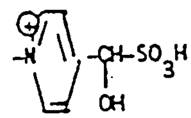 ,

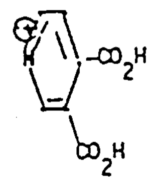 or 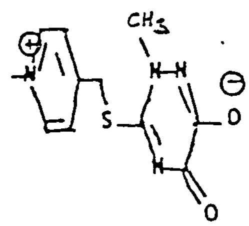 ,

by a process analogous to processes A to C as described in claim 1.

**5.** A process of claim for preparing a compound having the formula:

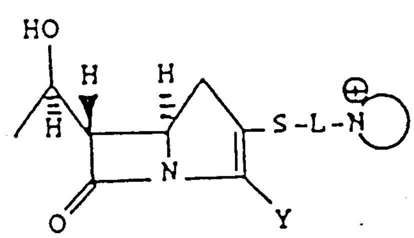

I

wherein L is $-CH_2CH_2-$, Y is $-CO_2Na$ and

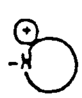

is

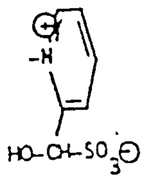 , 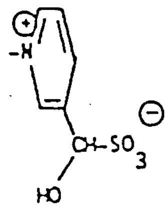 , 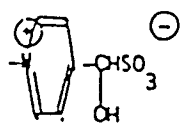 or 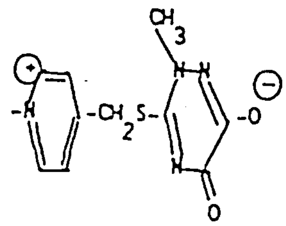

6. A process according to any one of claims 1 to 5, wherein the compound obtained is additionally combined with a DHP inhibitor, and, optionally, with a pharmaceutically acceptable carrier.

7. The process according to claim 6 wherein the DHP inhibitor is 7-(L-2-amino-2-carboxyethylthio)-2-(2,2-dimethylcyclopropanecarboxamide)-2-heptenoic acid.

**Patentansprüche**
**Patentansprüche für folgende Vertragsstaaten : BE, CH, DE, FR, GB, IT, LI, LU, NL, SE**

1. Verbindung der Formel:

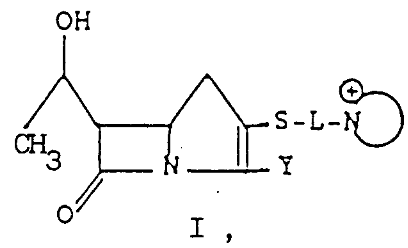

I ,

worin

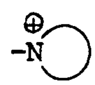

für

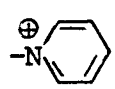

steht und worin die Pyridiniumgruppe durch:

A: eine saure Seitenkette der Stuktur A oder $-(CH_2)_n-X-(CH_2)_m-Y'-A$ substituiert sein muß, worin:

- n von 0-4 beträgt
- m von 0 bis 4 beträgt
- X $CHR^s$, CH = CH, Phenylen ($-C_6H_4-$), NH, N($C_1$-$C_4$-Alkyl), O, S, S = O, C = O, $SO_2$, $SO_2NH$, $CO_2$, CONH, $OCO_2$, OC = O, NHC = O bedeutet,
- $R^s$ H, O($C_1$-$C_4$-Alkyl), $NH_2$, NH($C_1$-$C_4$-Alkyl), N($C_1$-$C_4$-Alkyl)$_2$, CN, $CONH_2$, CON($C_1$-$C_4$-Alkyl)$_2$, $CO_2H$, $SO_2NH_2$, $SO_2NH$($C_1$-$C_4$-Alkyl) darstellt,
- Y' eine Einfachbindung, NH, N($C_1$-$C_4$-Alkyl), O, S ist,
- A eine saure Funktion darstellt, welche unter Carboxy ($CO_2H$), Phosphono [$P = O(OH)_2$], Alkylphosphono {P = O(OH)-[O($C_1$-$C_4$-Alkyl)]}, Alkylphosphinyl [P = O(OH)-($C_1$-$C_4$-Alkyl)], Phosphoramido [P = O(OH)--NH($C_1$-$C_4$-Alkyl) und $P = O(OH)NHR^x$], Sulfino ($SO_2H$), Sulfo ($SO_3H$), 5-Tetrazolyl ($CN_4H$), Heteroarylsulfonamido ($SO_2NHR^x$) und Acylsulfonamiden, die durch die Strukteren $CONHSO_2$($C_1$-$C_4$-Alkyl), $CONHSO_2N$($C_1$-$C_4$-Alkyl)$_2$, $SO_2NHCO$($C_1$-

$C_4$-Alkyl) und $SO_2NHCOR^x$ repräsentiert werden, ausgewählt ist,

$R^x$ eine monocyclische oder bicyclische Heteroarylgruppe darstellt, welche (a) wenn sie monocyclisch ist, bis zu 3 Heteroatome und bis zu insgesamt 6 Ringatome enthält, oder (b) wenn sie bicyclisch ist, bis zu 5 Heteroatome und 9-10 Ringatome enthält, worin die Heteroatome unter Stickstoff, Sauerstoff und Schwefel ausgewählt sind;

und wahlweise durch

B: ein Halogenatom substituiert ist;

L ein geradkettiges oder verzweigtkettiges, zweiwertiges $C_1$-$C_6$-Alkylen, $C_{2-6}$-Alkenylen oder $C_3$-$C_6$-Cycloalkylen oder -$C_1$-$C_4$-Alkylen-X-$C_1$-$C_4$-alkylen darstellt, worin X für O, S, NH oder N($C_1$-$C_4$-Alkyl) steht;

und

Y

i) -COOH, einen pharmazeutisch annehmbaren Ester oder ein Salz hievon;

ii) COOR, worin R eine entfernbare Carboxyschutzgruppe ist;

iii) COOM, worin M ein Alkalimetall bedeutet; oder

iv) $COO^-$ darstellt, mit der Maßgabe, daß, wenn Y eine andere Bedeutung als iv) besitzt, ein Gegenion $Z^{\ominus}$ vorhanden ist.

**2.** Verbindung nach Anspruch 1, worin die Verbindung ein Mitglied darstellt, welches von der Gruppe ausgewählt ist, die besteht aus:

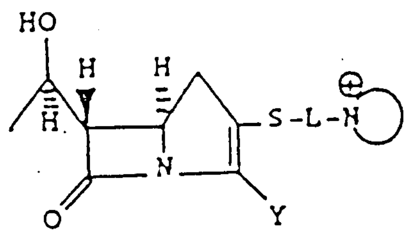

I

| Verbindung Nr. | L | 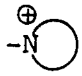 | Y |
|---|---|---|---|
| 1 | $-CH_2CH_2-$ | 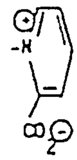 | $-CO_2Na$ |
| 2 | $-CH_2CH_2-$ | 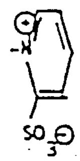 | $-CO_2Na$ |
| 3 | $-CH_2CH_2-$ | 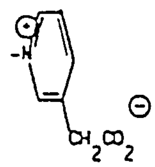 | $-CO_2Na$ |
| 4 | $-CH_2CH_2-$ | 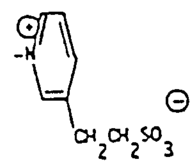 | $-CO_2Na$ |
| 5 | $-CH_2CH_2-$ | 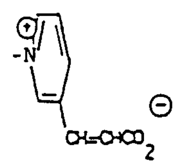 | $-CO_2Na$ |
| 6 | $-CH_2CH_2-$ | 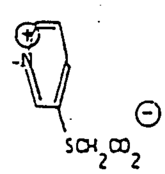 | $-CO_2Na$ |
| 7 | $-CH_2CH_2-$ | 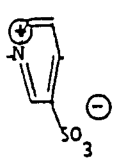 | $-CO_2Na$ |

| Verbindung Nr. | L | 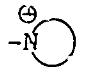 | Y |
|---|---|---|---|
| 8 | $-CH_2CH_2-$ | 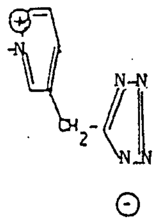 | $-CO_2Na$ |
| 9 | $-CH_2CH_2-$ | 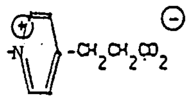 | $-CO_2Na$ |
| 10 | $-CH_2CH_2-$ | 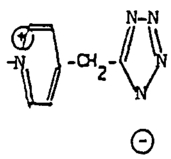 | $-CO_2Na$ |
| 11 | $-CH_2CH_2-$ | 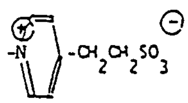 | $-CO_2Na$ |
| 12 | $-CH_2CH_2-$ | 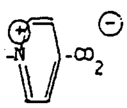 | $-CO_2Na$ |
| 13 | $-CH_2CH_2-$ | 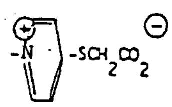 | $-CO_2Na$ |
| 14 | $-CH(CH_3)CH_2-$ | 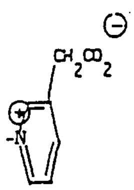 | $-CO_2Na$ |
| 15 | $-CH(CH_3)CH_2-$ | 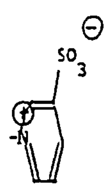 | $-CO_2Na$ |

| Verbindung Nr. | L | 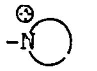 | Y |
|---|---|---|---|
| 16 | $-CH(CH_3)CH_2-$ | 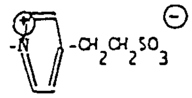 | $-CO_2Na$ |
| 17 | $-CH(CH_3)CH_2-$ | 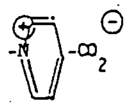 | $-CO_2Na$ |
| 18 | $-CH_2CH_2-$ | 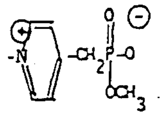 | $-CO_2Na$ |
| 19 | $-CH_2CH_2-$ | 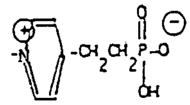 | $-CO_2Na$ |
| 20 | $-CH_2CH_2-$ | 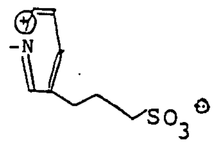 | $-CO_2Na$ |
| 21 | $-CH_2CH_2-$ | 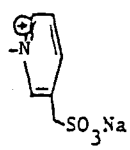 | $-CO_2^{\ominus}$ |
| 22 | $-CH_2CH_2-$ | 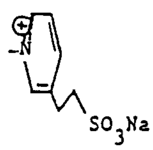 | $-CO_2^{\ominus}$ |
| 23 | $-CH_2CH_2-$ | 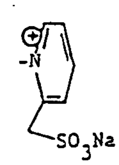 | $-CO_2^{\ominus}$ |
| 24 | $-CH_2CH_2-$ | 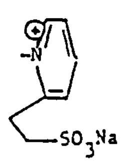 | $-CO_2^{\ominus}$ |

| Verbindung Nr. | L | 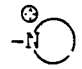 | Y |
|---|---|---|---|
| 25 | $-CH_2CH_2-$ | 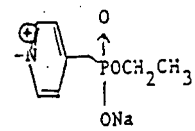 | $-CO_2^{\ominus}$ |
| 26 | $-CH_2CH_2-$ | 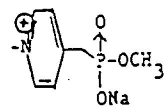 | $-CO_2^{\ominus}$ |
| 27 | $-CH_2CH_2-$ | 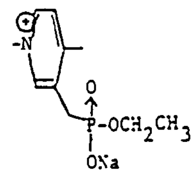 | $-CO_2^{\ominus}$ |
| 28 | $-CH_2CH_2-$ | 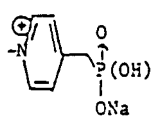 | $-CO_2^{\ominus}$ |
| 29 | $-CH_2CH_2-$ | 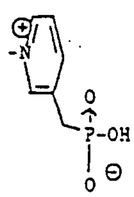 | $-CO_2^{\ominus}$ |
| 30 | $-CH_2CH_2-$ | 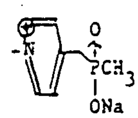 | $-CO_2^{\ominus}$ |
| 31 | $-CH_2CH_2-$ | 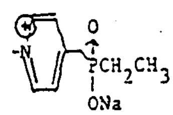 | $-CO_2^{\ominus}$ |
| 32 | $-CH_2CH_2-$ | 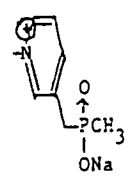 | $-CO_2^{\ominus}$ |

| Verbindung Nr. | L | 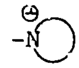 | Y |
|---|---|---|---|
| 33 | $-CH_2CH_2-$ | 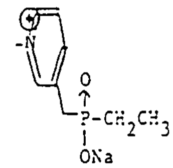 | $-CO_2^{\ominus}$ |
| 34 | $-CH_2CH_2-$ | 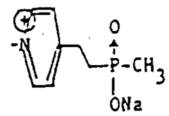 | $-CO_2^{\ominus}$ |
| 35 | $-CH_2CH_2-$ | 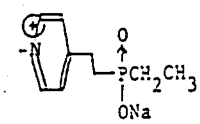 | $-CO_2^{\ominus}$ |
| 36 | $-CH_2CH_2-$ | 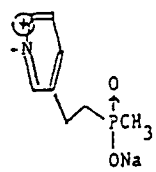 | $-CO_2^{\ominus}$ |
| 37 | $-CH_2CH_2-$ | 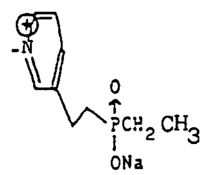 | $-CO_2^{\ominus}$ |
| 38 | $-CH_2CH_2-$ | 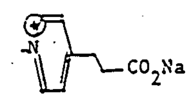 | $-CO_2^{\ominus}$ |
| 39 | $-CH_2CH_2-$ | 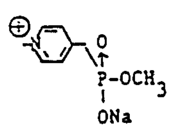 | $-CO_2^{\ominus}$ |
| 40 | $-CH_2CH_2-$ | 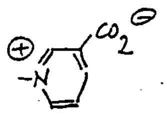 | $-CO_2Na$ |
| 41 | $-CH_2CH_2-$ | 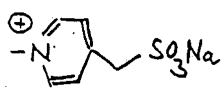 | $-CO_2^{\ominus}$ |

**3.** Verbindung nach Anspruch 1 mit der Struktur:

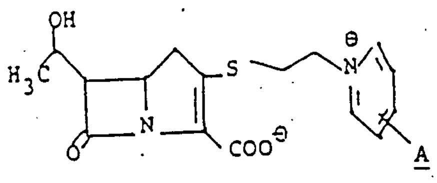
,

worin A eine saure Seitenkette der Struktur $-(CH_2)_n-X-(CH_2)_m-Y^1-A$ bedeutet, wie sie in Anspruch 1 definiert ist.

**4.** Verbindung der Formel:

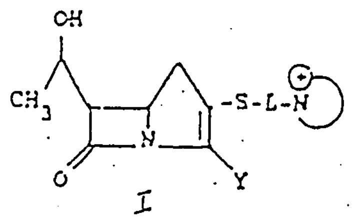
,

I

worin L und Y wie in Anspruch 1 definiert sind und

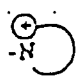 für 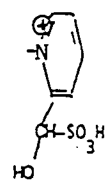 , 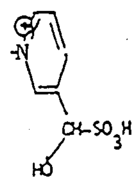 , 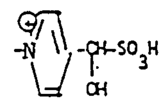 ,

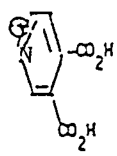 oder 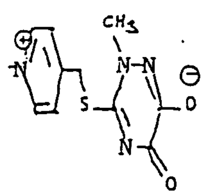

steht.

**5.** Verbindung nach Anspruch 4 mit der Formel:

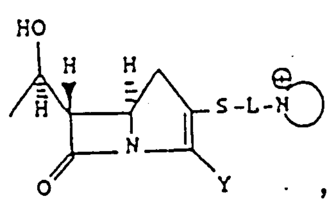
,

I

worin L $-CH_2CH_2-$ bedeutet, X $-CO_2Na$ darstellt und

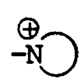

für

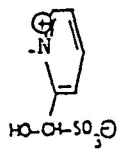 , 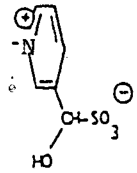 , 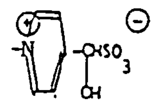 oder 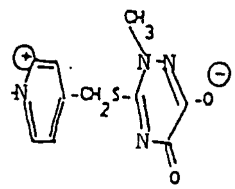

steht.

**6.** Kombination aus einer Verbindung nach eine der Ansprüche 1 bis 5 und eine DHP-Inhibitor.

**7.** Kombination nach Anspruch 6, worin der DHP-Inhibitor 7-(L-2-Amino--2-carboxyethylthio)-2-(2,2-dimethylcyclopropancarboxamid)-2-heptensäure ist.

**8.** Pharmazeutische Zusammensetzung zur antibiotischen Verwendung, umfassend eine antibakteriell wirksame Menge einer Verbindung nach einem der Ansprüche 1 bis 5, eine inhibierend wirksame Menge eines DHP-Inhibitors und wahlweise einen pharmazeutisch annehmbaren Trägere.

**Patentansprüche für folgenden Vertragsstaat : AT**

**1.** Verfahren zur Herstellung einer Verbindung der Formel:

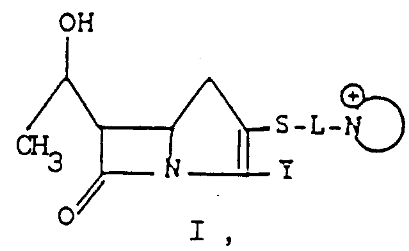

I ,

worin

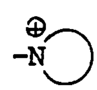

für

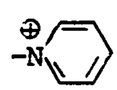

steht und worin die Pyridiniumgruppe durch:

A: eine saure Seitenkette der Struktur A oder $-(CH_2)_n-X-(CH_2)_m-Y'-A$ substituiert sein muß, worin:

- n von 0-4 beträgt
- m von 0 bis 4 beträgt
- X $CHR^s$, CH = CH, Phenylen ($-C_6H_4-$), NH, N($C_1$-$C_4$-Alkyl), O, S, S = O, C = O, $SO_2$, $SO_2NH$, $CO_2$, CONH, $OCO_2$, OC = O, NHC = O bedeutet,
- $R^s$ H, O($C_1$-$C_4$-Alkyl), $NH_2$, NH($C_1$-$C_4$-Alkyl), N($C_1$-$C_4$-Alkyl)$_2$, CN, $CONH_2$, CON($C_1$-$C_4$-Alkyl)$_2$, $CO_2H$, $SO_2NH_2$, $SO_2NH$($C_1$-$C_4$-Alkyl) darstellt,
- Y' eine Einfachbindung, NH, N($C_1$-$C_4$-Alkyl), O, S ist,

A eine saure Funktion darstellt, welche unter Carboxy ($CO_2H$), Phosphono [$P=O(OH)_2$], Alkylphosphono {$P=O(OH)$-[$O(C_1$-$C_4$-Alkyl)]}, Alkylphosphinyl [$P=O(OH)$-($C_1$-$C_4$-Alkyl)], Phosphoramido [$P=O(OH)$--$NH(C_1$-$C_4$-Alkyl) und $P=O(OH)NHR^x$], Sulfino ($SO_2H$), Sulfo ($SO_3H$), 5-Tetrazolyl ($CN_4H$), Heteroarylsulfonamido ($SO_2NHR^x$) und Acylsulfonamiden, die durch die Strukturen $CONHSO_2(C_1$-$C_4$-Alkyl), $CONHSO_2N(C_1$-$C_4$-Alkyl$)_2$, $SO_2NHCO(C_1$-$C_4$-Alkyl) und $SO_2NHCOR^x$ repräsentiert werden, ausgewählt ist,

$R^x$ eine monocyclische oder bicyclische Heteroarylgruppe darstellt, welche (a) wenn sie monocyclisch ist, bis zu 3 Heteroatome und bis zu insgesamt 6 Ringatome enthält, oder (b) wenn sie bicyclisch ist, bis zu 5 Heteroatome und 9-10 Ringatome enthält, worin die Heteroatome unter Stickstoff, Sauerstoff und Schwefel ausgewählt sind;

und wahlweise durch

B: ein Halogenatom substituiert ist;

L ein geradkettiges oder verzweigtkettiges, zweiwertiges $C_1$-$C_6$-Alkylen, $C_2$-$C_6$-Alkenylen oder $C_3$-$C_6$-Cycloalkylen oder -$C_1$-$C_4$-Alkylen-X-$C_1$-$C_4$-alkylen darstellt, worin X für O, S, NH oder $N(C_1$-$C_4$-Alkyl) steht;
und

Y

i) -COOH, einen pharmazeutisch annehmbaren Ester oder ein Salz hievon;
ii) COOR, worin R eine entfernbare Carboxyschutzgruppe ist;
iii) COOM, worin M ein Alkalimetall bedeutet; oder
iv) $COO^-$ darstellt, mit der Maßgabe, daß, wenn Y eine andere

Bedeutung als iv) besitzt, ein Gegenion $Z^\ominus$ vorhanden ist, welches:

A. das Umsetzen einer Verbindung der Formel

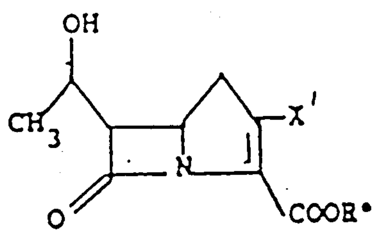

mit einer Verbindung der Formel

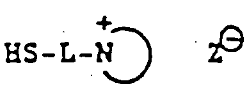

in Gegenwart einer Base und gegebenenfalls das Abspalten der blockierenden Gruppe in der entstehenden Verbindung der Formel

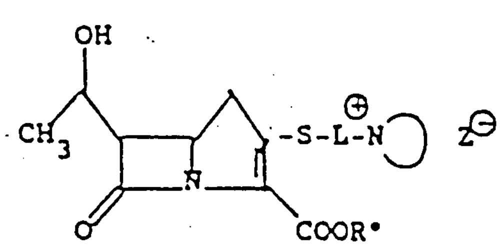

,

um

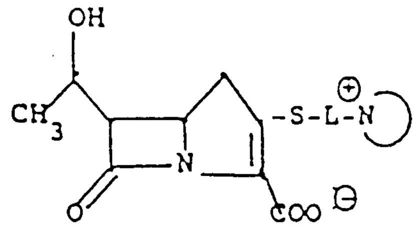

zu erhalten, worin $R^{o}$ eine andere Bedeutung als (-) oder H besitzt und eine leicht entfernbare blockierende Gruppe darstellt, und X' eine Leaving-Gruppe bedeutet; oder

B. das Umsetzen einer Verbindung der Formel

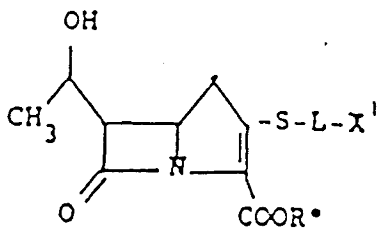

,

worin X' eine Leaving-Gruppe bedeutet und $R^{o}$ wie vorstehend definiert ist, mit

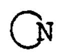

in Gegenwart einer Base oder von $Ag^{\oplus}$, und gegebenenfalls das Abspalten der blockierenden Gruppe in der entstehenden Verbindung der Formel

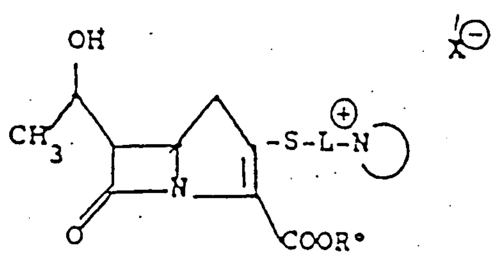

,

um

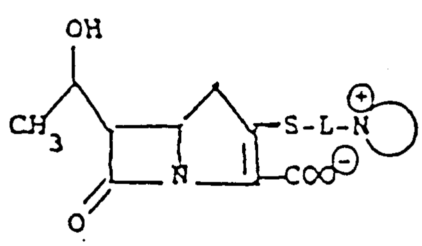

zu erhalten; oder

C. das Umsetzen der Verbindung der Formel

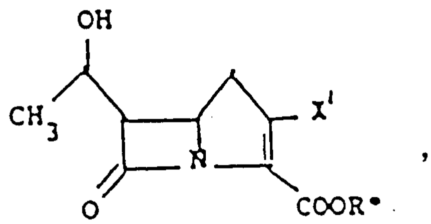

worin R° , X' und Z wie vorstehend definiert sind und X'' eine Leaving-Gruppe darstellt, mit NaHS; das Umsetzen der entstehenden Verbindung der Formel

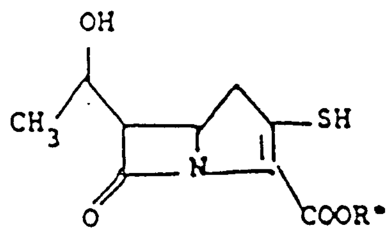

mit

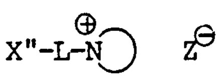

und gegebenenfalls das Abspalten der blockierenden Gruppe in der entstehenden Verbindung der Formel

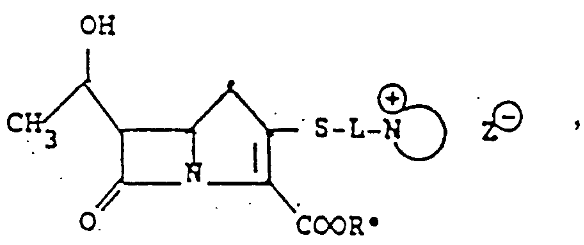

um

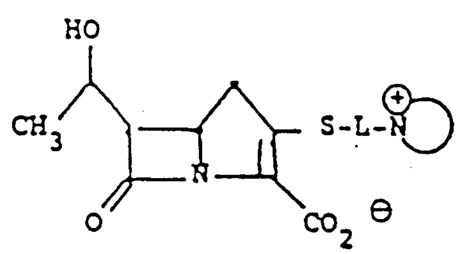

zu erhalten, umfaßt.

2. Verfähren nach Anspruch 1 zur Herstellung einer Verbindung, welche ein Mitglied darstellt, welches von der Gruppe ausgewählt ist, die besteht aus:

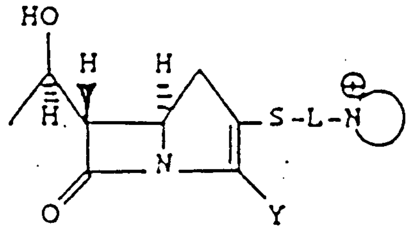

I

| Verbindung Nr. | L | 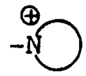 | Y |
|---|---|---|---|
| 1 | $-CH_2CH_2-$ | 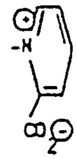 | $-CO_2Na$ |
| 2 | $-CH_2CH_2-$ | 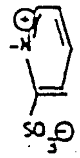 | $-CO_2Na$ |
| 3 | $-CH_2CH_2-$ | 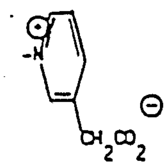 | $-CO_2Na$ |
| 4 | $-CH_2CH_2-$ | 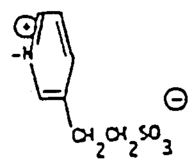 | $-CO_2Na$ |
| 5 | $-CH_2CH_2-$ | 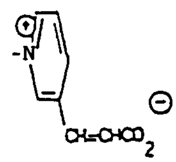 | $-CO_2Na$ |
| 6 | $-CH_2CH_2-$ | 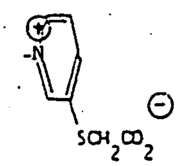 | $-CO_2Na$ |
| 7 | $-CH_2CH_2-$ | 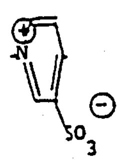 | $-CO_2Na$ |

| Verbindung Nr. | L | 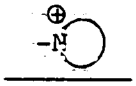 | Y |
|---|---|---|---|
| 8 | $-CH_2CH_2-$ | 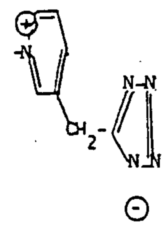 | $-CO_2Na$ |
| 9 | $-CH_2CH_2-$ | 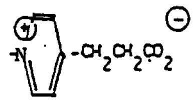 | $-CO_2Na$ |
| 10 | $-CH_2CH_2-$ | 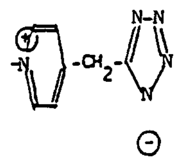 | $-CO_2Na$ |
| 11 | $-CH_2CH_2-$ | 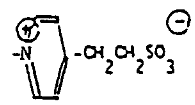 | $-CO_2Na$ |
| 12 | $-CH_2CH_2-$ | 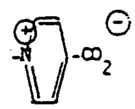 | $-CO_2Na$ |
| 13 | $-CH_2CH_2-$ | 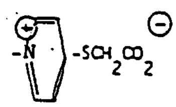 | $-CO_2Na$ |
| 14 | $-CH(CH_3)CH_2-$ | 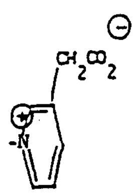 | $-CO_2Na$ |
| 15 | $-CH(CH_3)CH_2-$ | 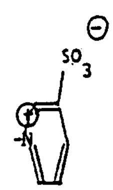 | $-CO_2Na$ |

| Verbindung Nr. | L | 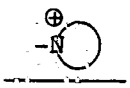 | Y |
|---|---|---|---|
| 16 | $-CH(CH_3)CH_2-$ | 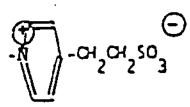 | $-CO_2Na$ |
| 17 | $-CH(CH_3)CH_2-$ | 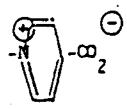 | $-CO_2Na$ |
| 18 | $-CH_2CH_2-$ | 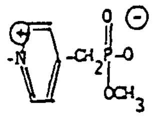 | $-CO_2Na$ |
| 19 | $-CH_2CH_2-$ | 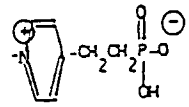 | $-CO_2Na$ |
| 20 | $-CH_2CH_2-$ | 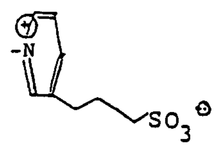 | $-CO_2Na$ |
| 21 | $-CH_2CH_2-$ | 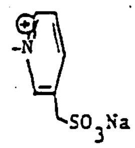 | $-CO_2^{\ominus}$ |
| 22 | $-CH_2CH_2-$ | 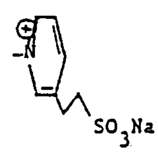 | $-CO_2^{\ominus}$ |
| 23 | $-CH_2CH_2-$ | 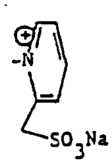 | $-CO_2^{\ominus}$ |
| 24 | $-CH_2CH_2-$ | 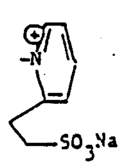 | $-CO_2^{\ominus}$ |

| Verbindung Nr. | L | 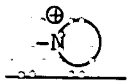 | Y |
|---|---|---|---|
| 25 | $-CH_2CH_2-$ | 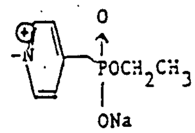 | $-CO_2^{\ominus}$ |
| 26 | $-CH_2CH_2-$ | 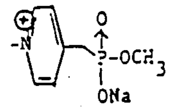 | $-CO_2^{\ominus}$ |
| 27 | $-CH_2CH_2-$ | 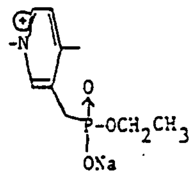 | $-CO_2^{\ominus}$ |
| 28 | $-CH_2CH_2-$ | 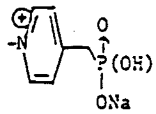 | $-CO_2^{\ominus}$ |
| 29 | $-CH_2CH_2-$ | 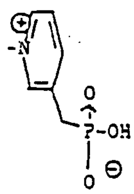 | $-CO_2^{\ominus}$ |
| 30 | $-CH_2CH_2-$ | 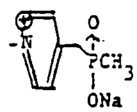 | $-CO_2^{\ominus}$ |
| 31 | $-CH_2CH_2-$ | 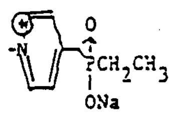 | $-CO_2^{\ominus}$ |
| 32 | $-CH_2CH_2-$ | 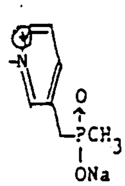 | $-CO_2^{\ominus}$ |

| Verbindung Nr. | L | 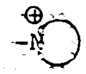 | Y |
|---|---|---|---|
| 33 | $-CH_2CH_2-$ | 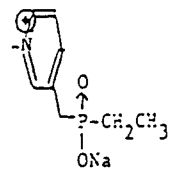 | $-CO_2^{\ominus}$ |
| 34 | $-CH_2CH_2-$ | 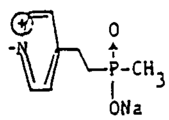 | $-CO_2^{\ominus}$ |
| 35 | $-CH_2CH_2-$ | 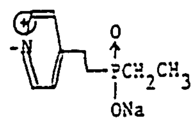 | $-CO_2^{\ominus}$ |
| 36 | $-CH_2CH_2-$ | 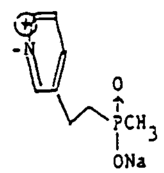 | $-CO_2^{\ominus}$ |
| 37 | $-CH_2CH_2-$ | 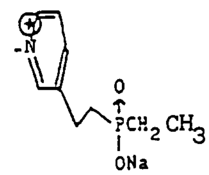 | $-CO_2^{\ominus}$ |
| 38 | $-CH_2CH_2-$ | 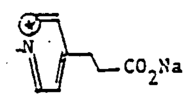 | $-CO_2^{\ominus}$ |
| 39 | $-CH_2CH_2-$ | 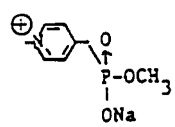 | $-CO_2^{\ominus}$ |
| 40 | $-CH_2CH_2-$ | 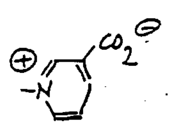 | $-CO_2Na$ |
| 41 | $-CH_2CH_2-$ | 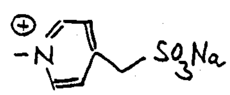 | $-CO_2^{\ominus}$ |

**3.** Verfahren nach Anspruch 1 zur Herstellung einer Verbindung mit der Struktur:

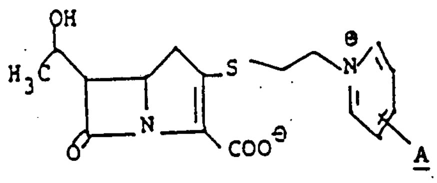

,

worin A eine saure Seitenkette der Struktur $-(CH_2)_n-X-(CH_2)_m-Y^1-A$ bedeutet, wie sie in Anspruch 1 definiert ist.

**4.** Verfahren zur Herstellung einer Verbindung der Formel:

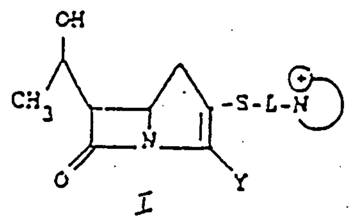

I ,

worin L und Y wie in Anspruch 1 definiert sind und

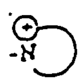 für 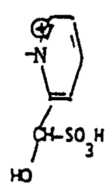 , 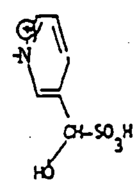 , 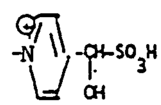 ,

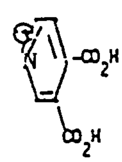 oder 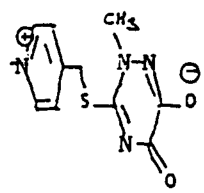

steht, durch ein Verfahren, welches zu den Verfahren A bis C, wie sie in Anspruch 1 definiert sind, analog ist.

**5.** Verfahren nach Anspruch 4 zur Herstellung einer Verbindung mit der Formel:

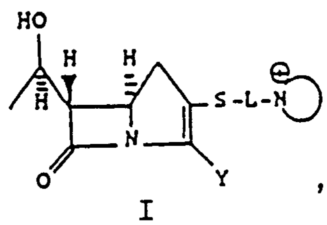

I ,

worin L $-CH_2CH_2-$ bedeutet, X $-CO_2Na$ darstellt und

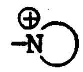

für

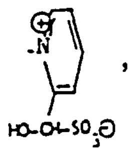 , 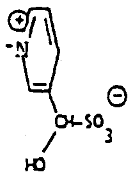 , 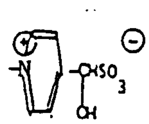 oder 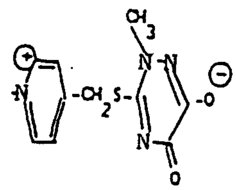

steht.

**6.** Verfahren nach einem der Ansprüche 1 bis 5, worin die erhaltene Verbindung zusätzlich mit einem DHP-Inhibitor und wahlweise mit einem pharmazeutisch annehmbaren Träger kombiniert wird.

**7.** Verfahren nach Anspruch 6, worin der DHP-Inhibitor 7-(L-2-Amino-2--carboxyethylthio)-2-(2,2-dimethyl-cyclopropancarboxamid)-2-heptensäure ist.

**Revendications**

**Revendications pour les Etats contractants suivants : BE, CH, DE, FR, GB, IT, LI, LU, NL, SE**

**1.** Composé de formule :

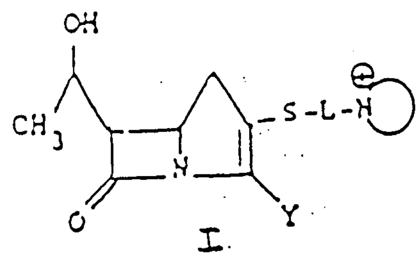

I.

dans laquelle

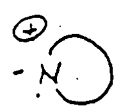

représente un groupe

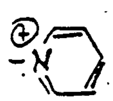 ,

et dans laquelle le groupe pyridinium doit être substitué avec :

A : une chaîne latérale acide de formule A ou - $(CH_2)_n$-X-$(CH_2)_m$-Y'-A dans laquelle :

n = 0 à 4

m = 0 à 4

X représente un groupe $CHR^s$, CH=CH, phénylène ($-C_6H_4-$), NH, N(alkyle en $C_1$-$C_4$), O, S,

$S=O$, $C=O$, $SO_2$, $SO_2NH$, $CO_2$, CONH, $OCO_2$, $OC=O$, $NHC=O$ ;

$R^s$ représente un atome d'hydrogène, un groupe O(alkyle en $C_1$-$C_4$), $NH_2$, NH(alkyle en $C_1$-$C_4$), N(alkyle en $C_1$-$C_4$)$_2$, CN, $CONH_2$, CON(alkyle en $C_1$-$C_4$)$_2$, $CO_2H$, $SO_2NH_2$, $SO_2NH$-(alkyle en $C_1$-$C_4$) ;

Y' représente une simple liaison, un groupe NH, N(alkyle en $C_1$-$C_4$), O, S ;

A représente une fonction acide choisie parmi les groupes carboxy ($CO_2H$), phosphono [$P=O(OH)_2$], alkylphosphono (P=O(OH)-[O(alkyle en $C_1$-$C_4$)]},alkylphosphinyle [P=O-(OH)-(alkyle en $C_1$-$C_4$)], phosphoramido [P=O(OH)-NH(alkyle en $C_1$-$C_4$) et P=O(OH)-NHR$^x$], sulfino ($SO_2H$), sulfo ($SO_3H$), 5-tétrazolyle ($CN_4H$), hétéroarylsulfonamido ($SO_2NHR^x$), et les groupes acylsulfonamide correspondant aux formules : $CONHSO_2$-(alkyle en $C_1$-$C_4$), $CONHSO_2N$(alkyle en $C_1$-$C_4$)$_2$, $SO_2NHCO$(alkyle en $C_1$-$C_4$) et SO-$_2NHCOR^x$ ;

$R^x$ représente un groupe hétéroaryle monocyclique ou bicyclique contenant (a), lorsqu'il est monocyclique, jusqu'à 3 hétéroatomes et jusqu'à 6 atomes de cycle au total, ou (b), lorsqu'il est bicyclique, jusqu'à 5 hétéroatomes et de 9 à 10 atomes de cycle, les hétéroatomes étant choisis parmi l'azote, l'oxygène et le soufre ; et éventuellement avec :

B : un atome d'halogène ;

L représente une chaîne linéaire ou ramifiée, un groupe alkylène en $C_1$-$C_6$ bivalent, un groupe alkénylène en $C_2$-$C_6$ ou cycloalkylène en $C_3$-$C_6$, ou un groupe (alkylène en $C_1$-$C_4$)-X-(alkylène en $C_1$-$C_4$) dans lequel X représente O, S, NH ou un groupe N(alkyle en $C_1$-$C_4$) ; et

Y représente :

i) un groupe -COOH, un ester ou un sel pharmaceutiquement acceptable de celui-ci ;
ii) un groupe COOR dans lequel R représente un groupe carboxy protecteur éliminable ;
iii) un groupe COOM dans lequel M représente un métal alcalin ; ou
iv) un groupe $COO^-$ à condition que lorsque Y ne correspond pas à la définition iv), un contre-ion $Z^-$ soit présent.

**2.** Composé selon la revendication 1, dans lequel le composé est choisi parmi le groupe suivant :

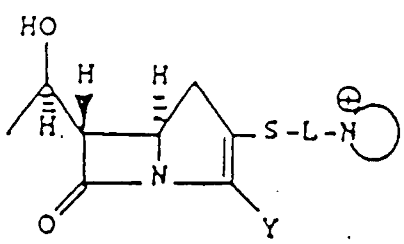

I

| Composé n° | L | 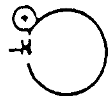 | Y |
|---|---|---|---|
| 1 | $-CH_2CH_2-$ | 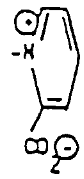 | $-CO_2Na$ |
| 2 | $-CH_2CH_2-$ | 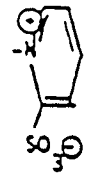 | $-CO_2Na$ |
| 3 | $-CH_2CH_2-$ | 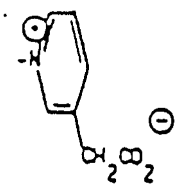 | $-CO_2Na$ |
| 4 | $-CH_2CH_2-$ | 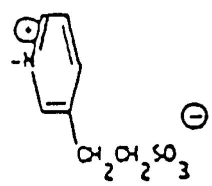 | $-CO_2Na$ |
| 5 | $-CH_2CH_2-$ | 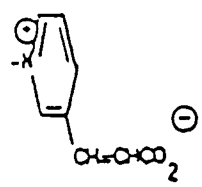 | $-CO_2Na$ |

| | | | |
|---|---|---|---|
| 6 | $-CH_2CH_2-$ | 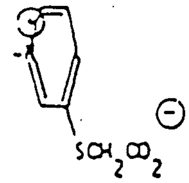 | $-CO_2Na$ |
| 7 | $-CH_2CH_2-$ | 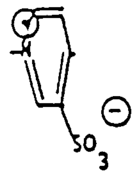 | $-CO_2Na$ |
| 8 | $-CH_2CH_2-$ | 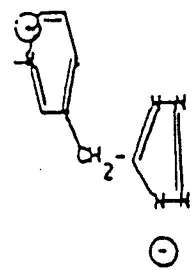 | $-CO_2Na$ |
| 9 | $-CH_2CH_2-$ | 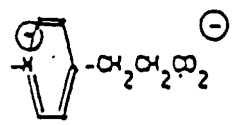 | $-CO_2Na$ |
| 10 | $-CH_2CH_2-$ | 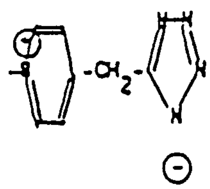 | $-CO_2Na$ |
| 11 | $-CH_2CH_2-$ | 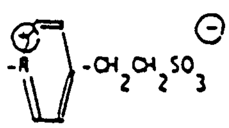 | $-CO_2Na$ |
| 12 | $-CH_2CH_2-$ | 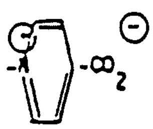 | $-CO_2Na$ |

| | | | |
|---|---|---|---|
| 13 | $-CH_2CH_2-$ | 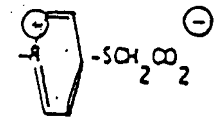 | $-CO_2Na$ |
| 14 | $-CH(CH_3)CH_2-$ | 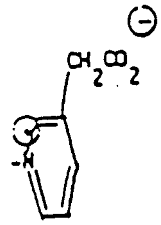 | $-CO_2Na$ |
| 15 | $-CH(CH_3)CH_2-$ | 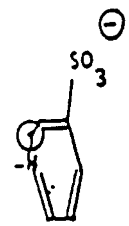 | $-CO_2Na$ |
| 16 | $-CH(CH_3)CH_2-$ | 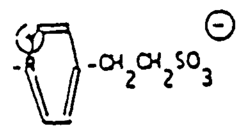 | $-CO_2Na$ |
| 17 | $-CH(CH_3)CH_2-$ | 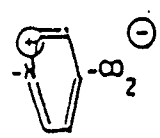 | $-CO_2Na$ |
| 18 | $-CH_2CH_2-$ | 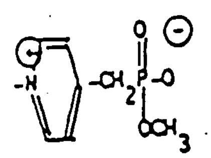 | $-CO_2Na$ |
| 19 | $-CH_2CH_2-$ | 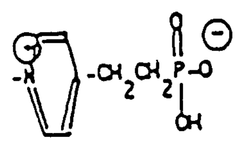 | $-CO_2Na$ |

| | | | |
|---|---|---|---|
| 20 | $-CH_2CH_2-$ | 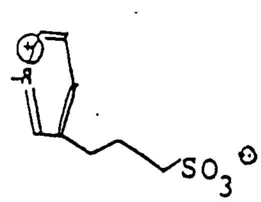 | $-CO_2Na$ |
| 21 | $-CH_2CH_2-$ | 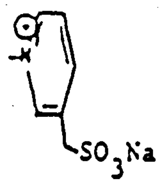 | $-CO_2^-$ |
| 22 | $-CH_2CH_2-$ | 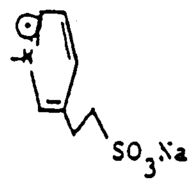 | $-CO_2^-$ |
| 23 | $-CH_2CH_2-$ | 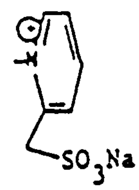 | $-CO_2^-$ |
| 24 | $-CH_2CH_2-$ | 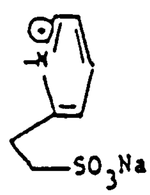 | $-CO_2^-$ |
| 25 | $-CH_2CH_2-$ | 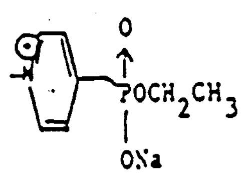 | $-CO_2^-$ |
| 26 | $-CH_2CH_2-$ | 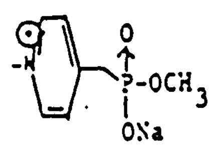 | $-CO_2^-$ |

| | | | |
|---|---|---|---|
| 27 | $-CH_2CH_2-$ | 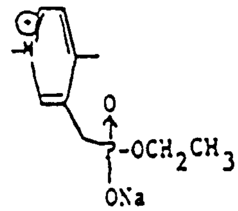 | $-CO_2^-$ |
| 28 | $-CH_2CH_2-$ | 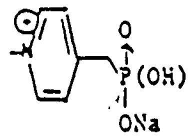 | $-CO_2^-$ |
| 29 | $-CH_2CH_2-$ | 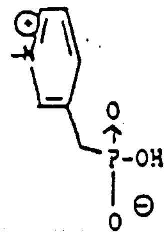 | $-CO_2^-$ |
| 30 | $-CH_2CH_2-$ | 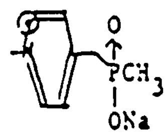 | $-CO_2^-$ |
| 31 | $-CH_2CH_2-$ | 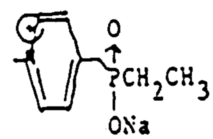 | $-CO_2^-$ |
| 32 | $-CH_2CH_2-$ | 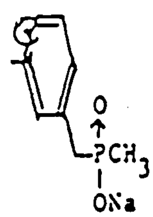 | $-CO_2^-$ |
| 33 | $-CH_2CH_2-$ | 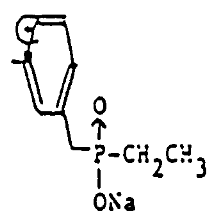 | $-CO_2^-$ |

| | | | |
|---|---|---|---|
| 34 | $-CH_2CH_2-$ | 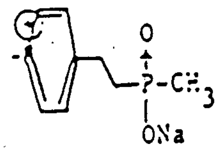 | $-CO_2^-$ |
| 35 | $-CH_2CH_2-$ | 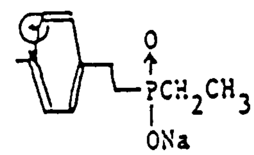 | $-CO_2^-$ |
| 36 | $-CH_2CH_2-$ | 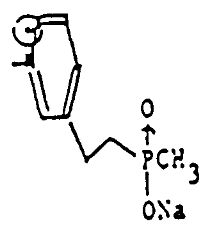 | $-CO_2^-$ |
| 37 | $-CH_2CH_2-$ | 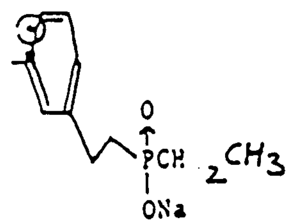 | $-CO_2^-$ |
| 38 | $-CH_2CH_2-$ | 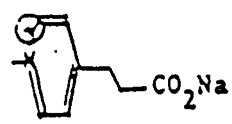 | $-CO_2^-$ |
| 39 | $-CH_2CH_2-$ | 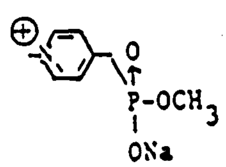 | $-CO_2^-$ |
| 40 | $-CH_2CH_2-$ | 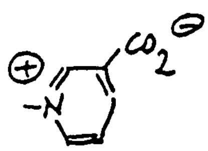 | $-CO_2Na$ |

| | | | |
|---|---|---|---|
| 41 | $-CH_2CH_2-$ | 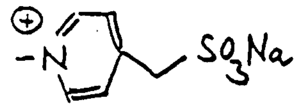 | $-CO_2^-$ |

3. Composé selon la revendication 1, de formule :

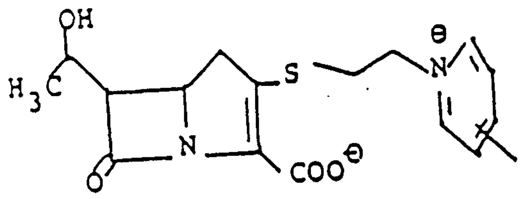

A

dans laquelle A représente une chaîne latérale acide de formule $-(CH_2)_n-X-(CH_2)_m-Y'-A$ telle que définie dans la revendication 1.

4. Composé de formule :

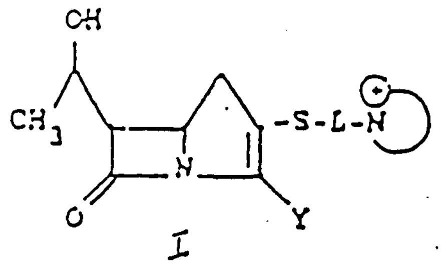

I

dans laquelle L et Y sont tels que définis dans le revendication 1, et

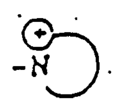

représente un groupe :

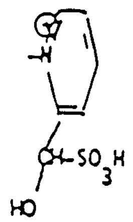 , 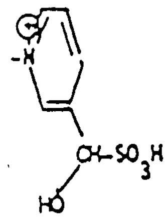 , 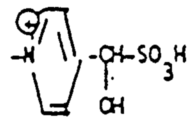 ,

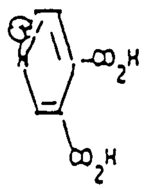 ou 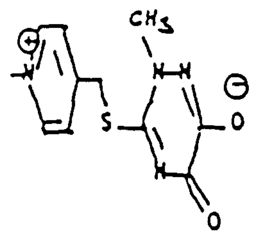 .

**5.** Composé selon la revendication 4, de formule :

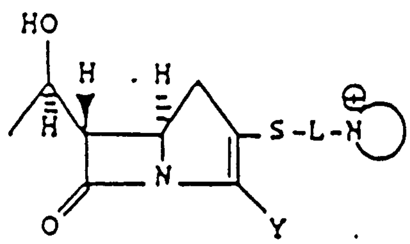 .

I

dans laquelle L représente un groupe $-CH_2CH_2-$, Y représente un groupe $-CO_2Na$, et

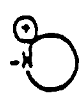 .

représente un groupe :

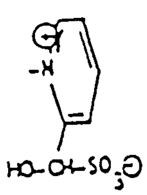 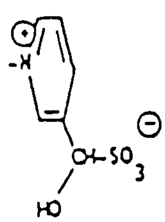 , 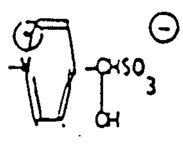 ou 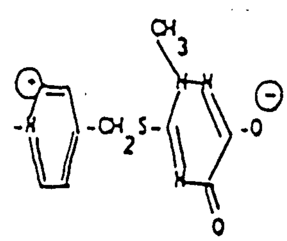

**6.** Composition contenant un composé selon l'une quelconque des revendications 1 à 5, et un inhibiteur de la DHP.

7. Composition selon la revendication 6, dans laquelle l'inhibiteur de la DHP est l'acide 7-(L-2-amino-2-carboxyéthylthio)-2-(2,2-diméthylcyclopropane carboxamide)-2-hepténoïque.

8. Composition pharmaceutique destinée à être utilisée comme antibiotique, comprenant une quantité à effet antibactérien d'un composé selon l'une quelconque des revendications 1 à 5, une quantité inhibitrice efficace d'un inhibiteur de la DHP, et éventuellement un véhicule pharmaceutiquement acceptable.

**Revendications pour l'Etat contractant suivant : AT**

1. Procédé de préparation d'un composé de formule :

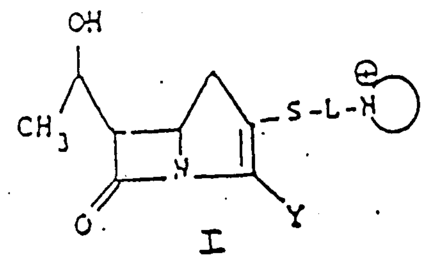

I

dans laquelle

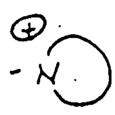

représente un groupe de formule

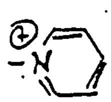

,

et dans laquelle le groupe pyridinium doit être substitué avec :

A : une chaîne latérale acide de formule A ou - $(CH_2)_n$-X-$(CH_2)_m$-Y'-A dans laquelle :

n = 0 à 4

m = 0 à 4

X représente un groupe $CHR^s$, CH = CH, phénylène ($-C_6H_4-$), NH, N(alkyle en $C_1$-$C_4$), O, S, S = O, C = O, $SO_2$, $SO_2NH$, $CO_2$, CONH, $OCO_2$, OC = O, NHC = O ;

$R^s$ représente un atome d'hydrogène, un groupe O(alkyle en $C_1$-$C_4$), $NH_2$, NH(alkyle en $C_1$-$C_4$), N(alkyle en $C_1$-$C_4$)$_2$, CN, $CONH_2$, CON(alkyle en $C_1$-$C_4$)$_2$, $CO_2H$, $SO_2NH_2$, $SO_2NH$-(alkyle en $C_1$-$C_4$) ;

Y' représente une simple liaison, un groupe NH, N(alkyle en $C_1$-$C_4$), O, S ;

A représente une fonction acide choisie parmi les groupes carboxy ($CO_2H$), phosphono [P = O$(OH)_2$], alkylphosphono (P = O(OH)-[O(alkyle en $C_1$-$C_4$)]},alkylphosphinyle (P = O-(OH)-(alkyle en $C_1$-$C_4$)], phosphoramido [P = O(OH)-NH(alkyle en $C_1$-$C_4$) et P = O(OH)-$NHR^x$], sulfino ($SO_2H$), sulfo ($SO_3H$), 5-tétrazolyle ($CN_4H$), hétéroarylsulfonamido ($SO_2NHR^x$), et les groupes acylsulfonamide correspondant aux formules : $CONHSO_2$-(alkyle en $C_1$-$C_4$), $CONHSO_2N$(alkyle en $C_1$-$C_4$)$_2$, $SO_2NHCO$(alkyle en $C_1$-$C_4$) et $SO_2NHCOR^x$ ;

$R^x$ représente un groupe hétéroaryle monocyclique ou bicyclique contenant (a), lorsqu'il est monocyclique, jusqu'à 3 hétéroatomes et jusqu'à 6 atomes de cycle au total, ou (b), lorsqu'il est bicyclique, jusqu'à 5 hétéroatomes et de 9 à 10 atomes de cycle, les hétéroatomes étant choisis parmi l'azote, l'oxygène et le soufre ; et éventuellement avec

:

B : un atome d'halogène ;

L représente une chaîne linéaire ou ramifiée, un groupe alkylène bivalent en $C_1$-$C_6$, un groupe alkénylène en $C_2$-$C_6$ ou cycloalkylène en $C_3$-$C_6$, ou un groupe (alkylène en $C_1$-$C_4$)-X-(alkylène en $C_1$-$C_4$) dans lequel X représente O, S, NH ou un groupe N(alkyle en $C_1$-$C_4$) ; et

Y représente :

i) un groupe -COOH, un sel ou un ester pharmaceutiquement acceptable de celui-ci ;

ii) un groupe COOR dans lequel R représente un groupe carboxy protecteur éliminable ;

iii) un groupe COOM dans lequel M représente un métal alcalin ; ou

iv) un groupe $COO^-$ à condition que lorsque Y ne correspond pas à la définition iv), un contre-ion $Z^-$ soit présent ; selon lequel :

A. on fait réagir un composé de formule :

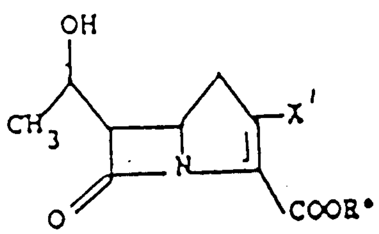

avec un composé de formule :

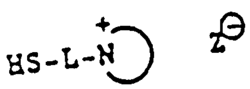

en présence d'une base, et on débloque éventuellement le composé résultant de formule :

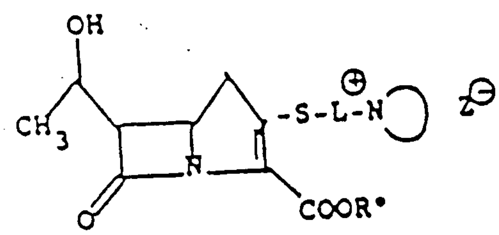

pour obtenir :

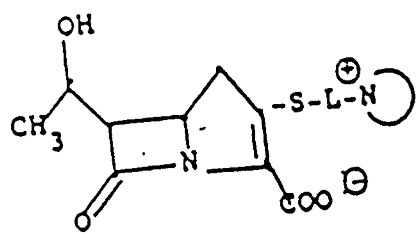

dans lequel R° est autre qu'une charge négative ou un atome d'hydrogène, et représente un groupe bloquant facilement éliminable, et X' représente un groupe partant ; ou

B. on fait réagir un composé de formule :

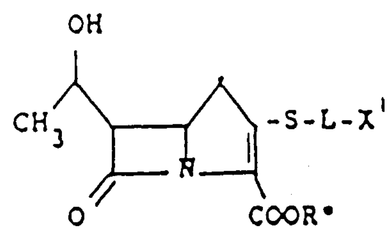

dans laquelle X' représente un groupe partant et R° est tel que défini ci-dessus, avec :

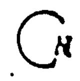

en présence d'une base ou d'ions $Ag^+$, et on débloque éventuellement le composé résultant de formule :

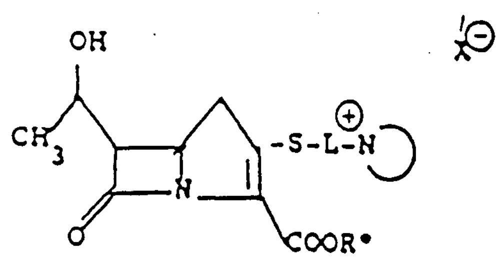

pour obtenir :

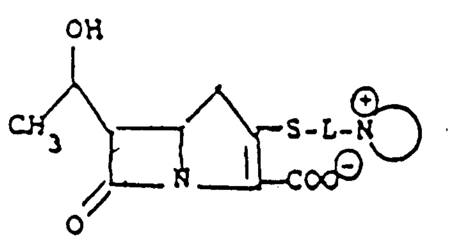

ou
C. on fait réagir le composé de formule :

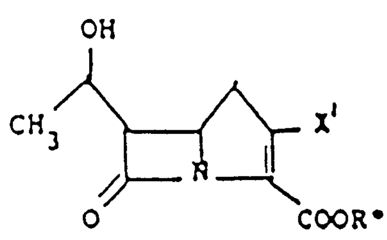

dans laquelle R°, X' et Z sont tels que définis ci-dessus, et X'' représente un groupe partant, avec

NaHS ;

on fait réagir le composé résultant de formule :

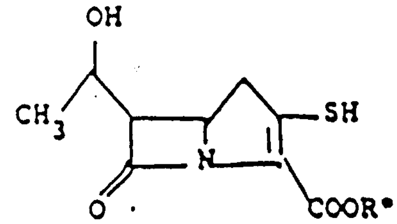

avec :

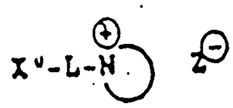

et on débloque éventuellement le composé résultant de formule :

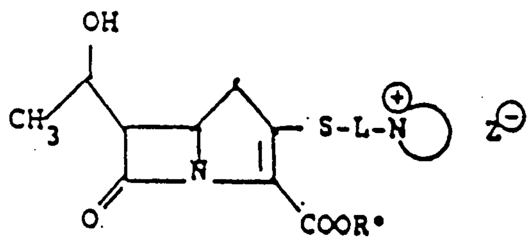

pour obtenir :

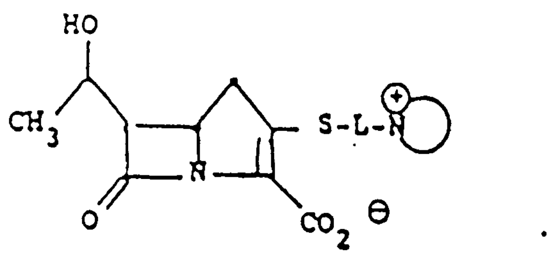

**2.** Procédé selon la revendication 1 de préparation d'un composé d'un groupe de composés comprenant :

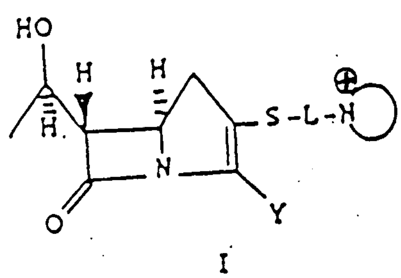

I

| Composé n° | L | 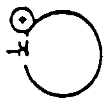 | Y |
| --- | --- | --- | --- |
| 1 | $-CH_2CH_2-$ | 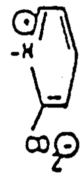 | $-CO_2Na$ |
| 2 | $-CH_2CH_2-$ | 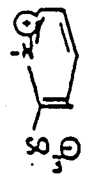 | $-CO_2Na$ |
| 3 | $-CH_2CH_2-$ | 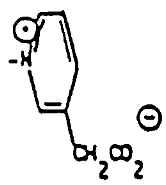 | $-CO_2Na$ |

| | | | |
|---|---|---|---|
| 4 | $-CH_2CH_2-$ | 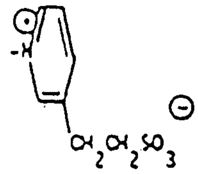 | $-CO_2Na$ |
| 5 | $-CH_2CH_2-$ | 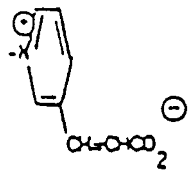 | $-CO_2Na$ |
| 6 | $-CH_2CH_2-$ | 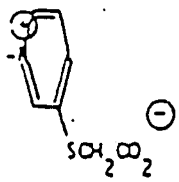 | $-CO_2Na$ |
| 7 | $-CH_2CH_2-$ | 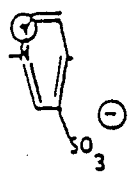 | $-CO_2Na$ |
| 8 | $-CH_2CH_2-$ | 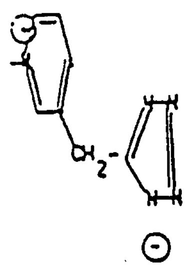 | $-CO_2Na$ |
| 9 | $-CH_2CH_2-$ | 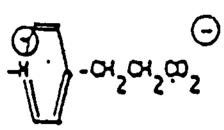 | $-CO_2Na$ |
| 10 | $-CH_2CH_2-$ | 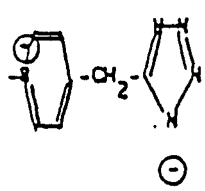 | $-CO_2Na$ |

| | | | |
|---|---|---|---|
| 11 | $-CH_2CH_2-$ | 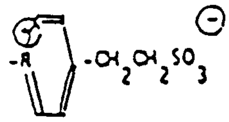 | $-CO_2Na$ |
| 12 | $-CH_2CH_2-$ | 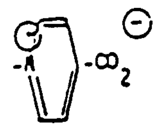 | $-CO_2Na$ |
| 13 | $-CH_2CH_2-$ | 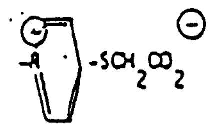 | $-CO_2Na$ |
| 14 | $-CH(CH_3)CH_2-$ | 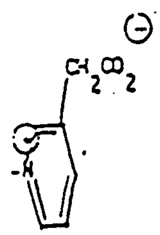 | $-CO_2Na$ |
| 15 | $-CH(CH_3)CH_2-$ | 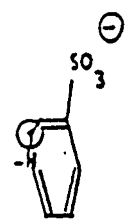 | $-CO_2Na$ |
| 16 | $-CH(CH_3)CH_2-$ | 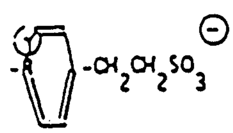 | $-CO_2Na$ |
| 17 | $-CH(CH_3)CH_2-$ | 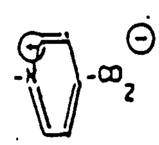 | $-CO_2Na$ |

| | | | |
|---|---|---|---|
| 18 | $-CH_2CH_2-$ | 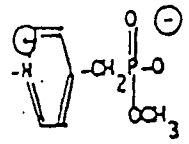 | $-CO_2Na$ |
| 19 | $-CH_2CH_2-$ | 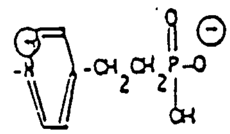 | $-CO_2Na$ |
| 20 | $-CH_2CH_2-$ | 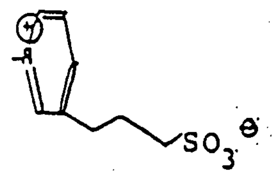 | $-CO_2Na$ |
| 21 | $-CH_2CH_2-$ | 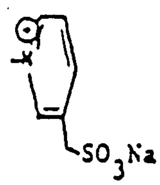 | $-CO_2^-$ |
| 22 | $-CH_2CH_2-$ | 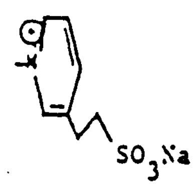 | $-CO_2^-$ |
| 23 | $-CH_2CH_2-$ | 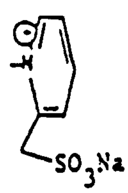 | $-CO_2^-$ |
| 24 | $-CH_2CH_2-$ | 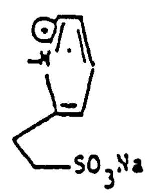 | $-CO_2^-$ |

| | | | |
|---|---|---|---|
| 25 | $-CH_2CH_2-$ | 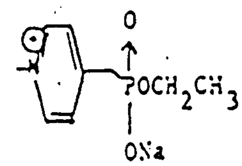 | $-CO_2^-$ |
| 26 | $-CH_2CH_2-$ | 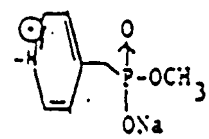 | $-CO_2^-$ |
| 27 | $-CH_2CH_2-$ | 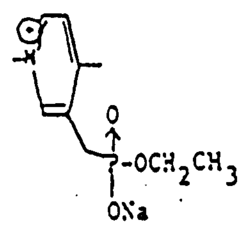 | $-CO_2^-$ |
| 28 | $-CH_2CH_2-$ | 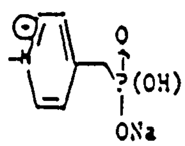 | $-CO_2^-$ |
| 29 | $-CH_2CH_2-$ | 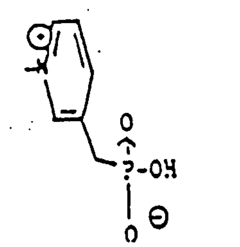 | $-CO_2^-$ |
| 30 | $-CH_2CH_2-$ | 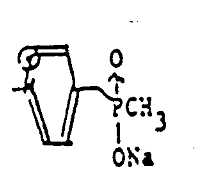 | $-CO_2^-$ |
| 31 | $-CH_2CH_2-$ | 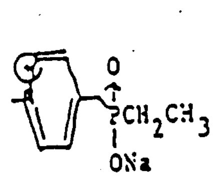 | $-CO_2^-$ |

| | | | |
|---|---|---|---|
| 32 | $-CH_2CH_2-$ | 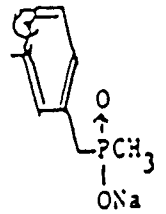 | $-CO_2^-$ |
| 33 | $-CH_2CH_2-$ | 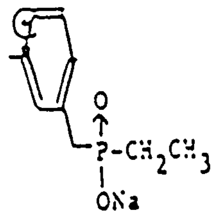 | $-CO_2^-$ |
| 34 | $-CH_2CH_2-$ | 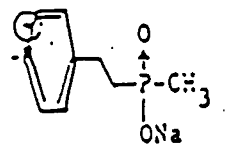 | $-CO_2^-$ |
| 35 | $-CH_2CH_2-$ | 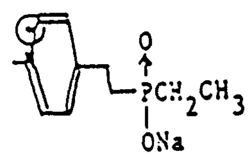 | $-CO_2^-$ |
| 36 | $-CH_2CH_2-$ | 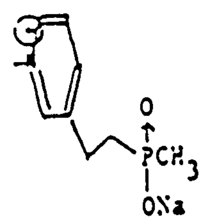 | $-CO_2^-$ |
| 37 | $-CH_2CH_2-$ | 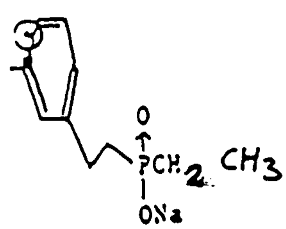 | $-CO_2^-$ |
| 38 | $-CH_2CH_2-$ | 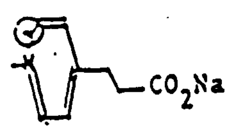 | $-CO_2^-$ |

| | | | |
|---|---|---|---|
| 39 | $-CH_2CH_2-$ | 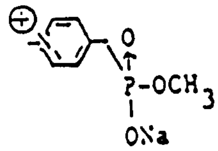 | $-CO_2^-$ |
| 40 | $-CH_2CH_2-$ | 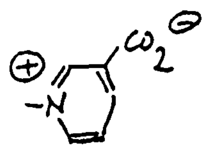 | $-CO_2Na$ |
| 41 | $-CH_2CH_2-$ | 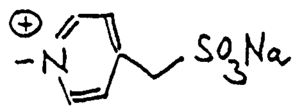 | $-CO_2^-$ |

**3.** Procédé selon la revendication 1, de préparation d'un composé de formule :

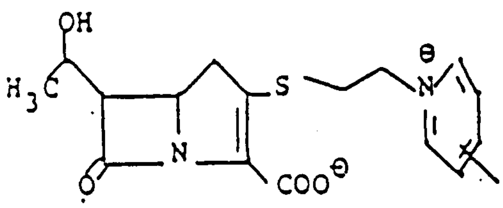

A

dans laquelle A représente une chaîne latérale acide de formule $-(CH_2)_n-X-(CH_2)_m-Y'-A$ telle que définie ci-dessus.

**4.** Procédé de préparation d'un composé de formule :

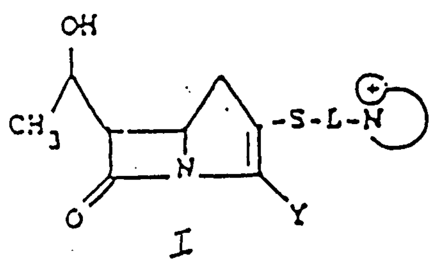

I

dans laquelle L et Y sont tels que définis dans la revendication 1, et

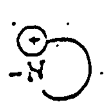

représente :

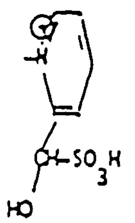 , 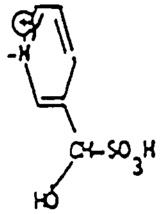 , 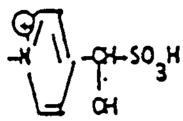 ,

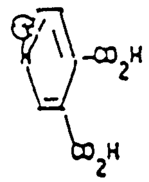 ou 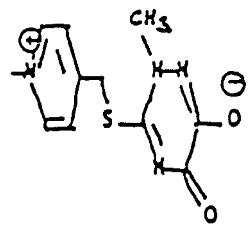 ,

selon un procédé analogue aux procédés A à C décrit dans la revendication 1.

**5.** Procédé selon la revendication 4, de préparation d'un composé de formule :

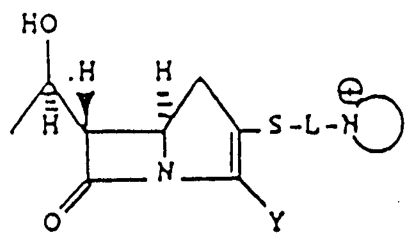

I

dans laquelle L représente un groupe $-CH_2CH_2-$, Y représente un groupe $-CO_2Na$, et

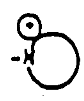

représente un groupe :

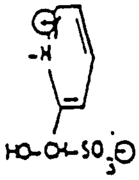 , 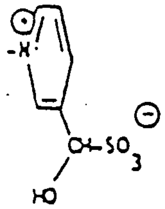 , 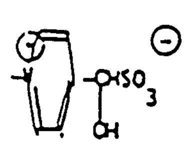 ou 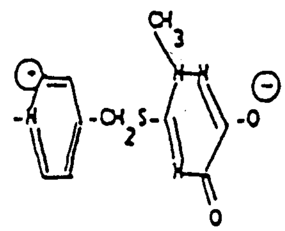

**6.** Procédé selon l'une quelconque des revendications 1 à 5, dans lequel le composé obtenu est en outre associé avec un inhibiteur de la DHP, et éventuellement avec un véhicule pharmaceutiquement acceptable.

**7.** Procédé selon la revendication 6, dans lequel l'inhibiteur de la DHP est l'acide 7-(L-2-amino-2-carboxyéthylthio)-2-(2,2-diméthylcyclopropane carboxamide)-2-hepténoïque.